# EUROPEAN PATENT SPECIFICATION

(11) **EP 4 504 095 B1**
(45) Date of publication and mention of the grant of the patent: **13.05.2026**
(21) Application number: 23717897.5
(22) Date of filing: 05.04.2023
(51) Int. Cl.: A61C 9/00

(54) **INTRAORAL SCANNING DEVICE WITH EXTENDED FIELD OF VIEW**
INTRAORALE SCANVORRICHTUNG MIT ERWEITERTEM SICHTFELD
DISPOSITIF DE BALAYAGE INTRA-ORAL DOTÉ D'UN CHAMP DE VISION ÉTENDU

(30) Priority: 08.04.2022 EP 22167462; 11.05.2022 EP 22172761; 22.02.2023 EP 23158001
(43) Date of publication of application: 12.02.2025
(73) Proprietor: 3Shape A/S, 1060 Copenhagen K (DK)
(72) Inventor: KJÆR, Rasmus, 1060 Copenhagen K (DK); ÖJELUND, Henrik, 1060 Copenhagen K (DK)
(74) Representative: Zacco Denmark A/S
(86) International application number: PCT/EP2023/058980
(87) International publication number: WO 2023/194460

(56) References cited:
- WO-A1-2011/118839
- WO-A1-2011/118839
- US-A1- 2019 388 193
- US-A1- 2019 388 193
- US-A1- 2020 205 942
- US-A1- 2020 205 942

## Description

### Technical field

The present disclosure relates to an intraoral scanning device for scanning a dental object. The disclosure further relates to a dental scanning system for acquiring images of the object and for generating a digital representation of the object. In particular, the present disclosure relates to an intraoral scanning device comprising a plurality of scan units.

### Background

Digital dentistry is increasingly popular and offers several advantages over non-digital techniques. Digital dental scanning systems typically utilize a scanning device, such as an intraoral 3D scanner, to generate a three-dimensional digital representation of an intraoral three-dimensional object/surface of a subject. A variety of different technologies exist within scanning devices, such as triangulation-based scanning, confocal scanning, focus scanning, x-ray scanning, and stereo vision.

Optical scanning devices often feature a projector unit for projecting an illumination pattern onto the surface of a 3D object, and an image sensor for acquiring one or more images of the illuminated object. For triangulation-based scanning devices, the projector unit and the camera are offset such that they form a triangle with a given point on the surface of the illuminated object.

In general, a scanning device is associated with a field of view (FOV). The FOV can be understood as the extent of the observable world that is seen at any given moment by the scanning device. Typically, the FOV is reported as an area measure at a given distance from the projector or camera, or the area can be measured a given distance below a tip of the scanning device.

Within digital dentistry, a full digital impression of a patient's teeth is typically obtained. Thus, during a scanning session, the dentist maneuvers the tip of the scanning device across the patient's dental arches until images of all the teeth are obtained. In some cases, only parts of the patient's dentition are imaged using the scanning device. However, in general it is desired that the digital impression is obtained fast and that the scanning is pleasant for the patient. Since the FOV determines how much of the teeth can be scanned by the scanning device at a given moment, the FOV consequently influences the time it takes to complete a scan.

It is generally desired to have a large FOV, thus covering a large area, since it allows for more data/images to be captured at any given moment, and thus enables a faster scan. Furthermore, a large FOV enables a larger overlap of the acquired data, which enables a more accurate and reliable generation of the digital 3D representation of the patient's teeth. In some cases, it can be difficult to image the patient's dentition if one or more teeth are missing. Hence, in such cases it is beneficial to have a scanning device with a large FOV, since it enables capturing images of the neighboring teeth across a missing tooth.

In addition to a large FOV, it is generally desired that the tip of the scanning device that enters the patient's mouth during the scanning session is rather compact or small, since large scanning tips can be unpleasant to the patient. Furthermore, if the tip of the scanning device is too large, such as if the tip height is too large, it can be difficult to reach all relevant areas inside the oral cavity of the patient. Therefore, it is desired to have a small tip height of the scanning device.

It is therefore of interest to provide a scanning device with a reduced tip height and a large field of view. A challenge associated herewith is that typically the field of view scales with the tip height. As an example, a scanning device having a very small tip, e.g. for use on a child, will generally have a smaller FOV compared to a regular sized tip, e.g. configured for adults. Therefore, it remains a challenge how to increase the FOV while keeping the tip height of the scanning device minimal.

US 2020/205942 A1 discloses a method of scanning an oral cavity including: acquiring, using an intraoral scanner (IOS) head, without changing a position of the IOS head, a first image of a first region of interest (ROI) and a second image of a second ROI where the first and the second ROIs are of different portions of a dental arch of the oral cavity and do not overlap; reconstructing depth information for the first and the second ROI; and generating a single model of the dental arch by combing the depth information.

### Summary

The invention is as defined in the appended claims.

There are further challenges with the above objective of providing a scanning device with a large field of view. In general, providing a large field of view using close-focus optics, i.e. optics with a high focusing power or short focal length, results in a large variation in magnification and illumination in the projected light / acquired images. Furthermore, it can result in a large angle of incidence of the projected light with a front window in the tip of the scanning device, which consequently results in unwanted reflections from said front window. Additionally, using close-focus optics can result in a large view angle between the camera(s) and the projector unit of the scanning device. Therefore, it is of interest to provide a scanning device with a large field of view, which overcomes the mentioned drawbacks of using close-focus optics.

The present disclosure addresses the above-mentioned challenges by providing a scanning device comprising one or more scan units, wherein each scan unit comprises at least one projector unit, and at least one camera, wherein at least one of the scan units further comprises a reflecting element. In preferred embodiments, the scanning device comprises two or more scan units e.g. arranged in series along a longitudinal axis of the scanning device.

The inventors have realized how to provide a scanning device, wherein the tip height of the scanning device can remain relatively low while the field of view is increased. This is achieved by providing a scanning device comprising a plurality of scan units, each scan unit comprising at least one projector unit and one or more cameras, wherein at least one scan unit further comprises a reflecting element, such as a mirror, configured to redirect the light from the projector unit and/or for redirecting reflected light from an illuminated surface of a scanned object. In particular, the inventors have realized that the field of view can be scaled with the number of scan units of the scanning device. Thus, the field of view of the scanning device can be extended by positioning multiple scan units in succession, such as in series, within the scanning device.

The present disclosure therefore relates to an intraoral scanning device for scanning a dental object, the scanning device comprising: an elongated probe; one or more scan units, each scan unit comprising at least one projector unit; and at least one camera comprising an image sensor for acquiring images, wherein at least one of the scan units further comprises a reflecting element. In some embodiments, the scanning device comprises at least two scan units.

In some embodiments, the scanning device comprises an elongated probe defining a longitudinal axis of the scanning device; at least two scan units, each scan unit comprising at least one projector unit configured to project a light pattern onto a surface of the dental object, wherein the projector unit defines a projector optical axis; and at least one camera comprising an image sensor for acquiring images, wherein the at least one camera defines a camera optical axis, wherein at least one of the scan units further comprises a reflecting element configured to reflect light from the projector unit(s) of that scan unit and/or configured to reflect light from the surface of the dental object and onto the image sensor(s) of each camera of that scan unit, wherein each scan unit defines a field of view (FOV).

In other embodiments, the scanning device comprises an elongated probe defining a longitudinal axis of the scanning device; at least two scan units, each scan unit comprising at least one projector unit configured to project a light pattern onto a surface of the dental object, wherein the projector unit defines a projector optical axis; and at least one camera comprising an image sensor for acquiring images, wherein the at least one camera defines a camera optical axis, wherein each scan unit further comprises a reflecting element. The reflecting element is configured to reflect light from the projector unit(s) of the given scan unit associated with the reflecting element and/or configured to reflect light from the surface of the dental object and onto the image sensor(s) of each camera of the given scan unit associated with the reflecting element, wherein each scan unit defines a field of view (FOV).

The scanning device disclosed herein has several advantages over existing scanning devices. One advantage is that incorporating multiple scan units in the scanning device allows to extend the total field of view, such that the intraoral scanning device has a field of view, which is larger than the FOV of each scan unit. Accordingly, the FOV of the scanning device can be scaled by the number of scan units of the device. A large FOV implies that the scanning device captures a larger extent of the object being scanned, e.g. the teeth/gums of a subject. Consequently, more data/images can be acquired per unit of time, which allows a 3D representation of the scanned object to be generated faster. Additionally, a large field of view allows for smaller registration errors and consequently a better global accuracy of the 3D representation. Registration may be understood as the process of determining where scan data, such as a scan patch (e.g. a point cloud), fits in the 3D representation. In other words, registration may be understood as determining the location and orientation of a given scan patch such that it can form part of the 3D representation of the dental object.

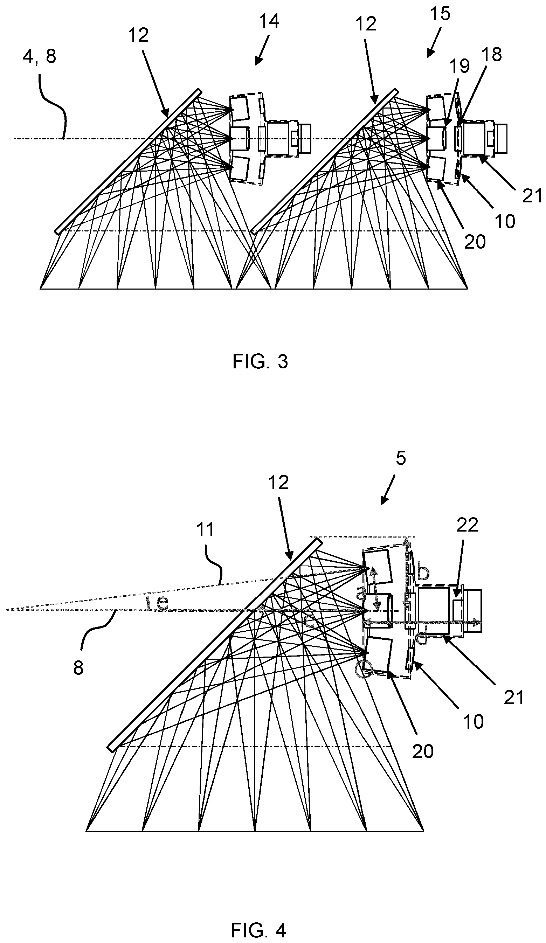

Another advantage of the aforementioned embodiment, wherein each scan unit comprises a reflecting element, is that the scanning device can be made rather compact, since the space in front of one reflecting element can be occupied at least partly by another scan unit. In particular, this is the case if the reflecting elements are mirrors, wherein a surface normal of each mirror defines an angle with respect to the projector optical axis of each projector unit of between 40° to 45°, such as approximately 45 degrees. Such an embodiment is shown in figure 3, where it is seen that a first scan unit overlaps the reflecting element of another scan unit placed behind the first. Thus, the multiple scan units can be positioned quite close as indicated in figure 3.

Another advantage of the presently disclosed scanning device is that the camera working distance is increased and the perspective is reduced, when at least one of the scan units further comprises a reflecting element, such as a mirror, as disclosed herein. In particular, this is the case if the projector unit and cameras of at least one scan unit are 'forward-looking', which may be understood such that the projector unit projects light towards the distal end of the elongated probe of the scanning device. In other words, a center beam of the projector unit of at least one scan unit is preferably substantially parallel with a longitudinal axis of the scanning device. Preferably, the reflecting element is then positioned in front of the scan unit such that the reflecting element is configured to direct the light from the projector unit towards the object to be scanned. Alternatively, the scan unit(s) may be oriented such that light from the projector unit is projected backwards, i.e. towards the proximal end of the scanning device. In that case, the reflecting element may similarly be positioned such that the reflecting element is configured to direct the light from the projector unit towards the object to be scanned. In other words, the reflecting element is preferably configured to change the direction of a center beam of the projected light from substantially parallel to the longitudinal axis of the scanning device to a direction forming an angle with said axis, such as a direction substantially orthogonal to the longitudinal axis of the scanning device. Thus, the working distance of the camera(s) is increased and the perspective is reduced, which has the following benefits:
- A smaller variation in magnification and illumination;
- A smaller angle of incidence with a front window(s) of the scanning device; and
- A smaller view angle between camera and projector.

The relative term 'smaller' should in this context be understood as when compared to a situation, where the scan unit does not comprise a reflecting element to change the direction of the projected light (in order to increase the working distance of the camera(s)). In such a situation, the scan unit is oriented such that the projector unit projects the light directly downwards, i.e. such that a center beam of the projected light is substantially orthogonal to the longitudinal direction of the scanning device. This configuration may also be referred to as 'downward-looking'. A drawback of using a scan unit which is configured as 'downward-looking' is that in order to keep the tip height reduced, close-focus optics is necessary, which implies that in order to get a decent field of view at a short focus distance, a large angular field of view (such as 90°) is needed, which consequently results in a large variation in magnification and illumination, a large angle of incidence with a front window, and a large view angle between camera(s) and projector unit(s). Accordingly, all of these drawbacks are overcome when providing the scanning device with a forward-looking scan unit comprising a reflecting element as described above. However, if a longer focus distance is feasible, a 'downward-looking' scan unit can be implemented in the scanning device without the aforementioned drawbacks becoming an issue.

Another challenge with a downward-looking scan unit is that the minimum tip height of the scanning device is limited by the minimum component height, i.e. the longitudinal dimension of the scan unit including the projector unit(s) and camera(s), and possibly projector and camera optics such as collimation lenses, and light source(s). The minimum achievable tip height of the scanning device is further limited by the minimum focus distance achieved by the scan unit. Thus, without the reflecting element, thereby forcing the projector unit(s) and camera(s) to have a short focus distance (close-focus optics), this results in the drawbacks mentioned above. On the contrary, if the scan unit is rotated such that it is forward-looking and combined with a reflecting element, the minimum tip height is limited by the size of the reflecting element and the lateral dimension(s) of the scan unit.

In yet another embodiment, the intraoral scanning device comprises an elongated probe defining a longitudinal axis of the scanning device; a first scan unit comprising: at least one projector unit configured to project a light pattern onto a surface of the dental object, wherein the projector unit defines a first projector optical axis; at least one camera comprising an image sensor for acquiring images, wherein the at least one camera defines a camera optical axis; and a reflecting element configured to reflect light from the projector unit(s) and/or reflect light from the surface of the dental object and onto the image sensor(s) of each camera of the scan unit. In this embodiment, the scanning device further comprises a second scan unit comprising: at least one projector unit configured to project a light pattern onto a surface of the dental object, wherein the projector unit defines a second projector optical axis; and at least one camera comprising an image sensor for acquiring images, wherein the at least one camera defines a camera optical axis, wherein each scan unit defines a field of view (FOV), and wherein the first projector optical axis and the second projector optical axis define an angle of at least 45°. In some embodiments, this angle is measured in a plane parallel to the longitudinal axis of the scanning device. In other embodiments, this angle is measured in a plane orthogonal to the longitudinal axis of the scanning device.

In yet another embodiment, the intraoral scanning device comprises an elongated probe defining a longitudinal axis of the scanning device; a first scan unit comprising: at least one projector unit configured to project a light pattern onto a surface of the dental object, wherein the projector unit defines a first projector optical axis which is substantially parallel to the longitudinal axis of the scanning device; at least one camera comprising an image sensor for acquiring images, wherein the at least one camera defines a camera optical axis; and a reflecting element configured to reflect light from the projector unit(s) and/or reflect light from the surface of the dental object and onto the image sensor(s) of each camera of the scan unit. In this embodiment, the scanning device further comprises a second scan unit comprising: at least one projector unit configured to project a light pattern onto a surface of the dental object, wherein the projector unit defines a second projector optical axis which is substantially orthogonal to the longitudinal axis of the scanning device; and at least one camera comprising an image sensor for acquiring images, wherein the at least one camera defines a camera optical axis, wherein each scan unit defines a field of view (FOV).

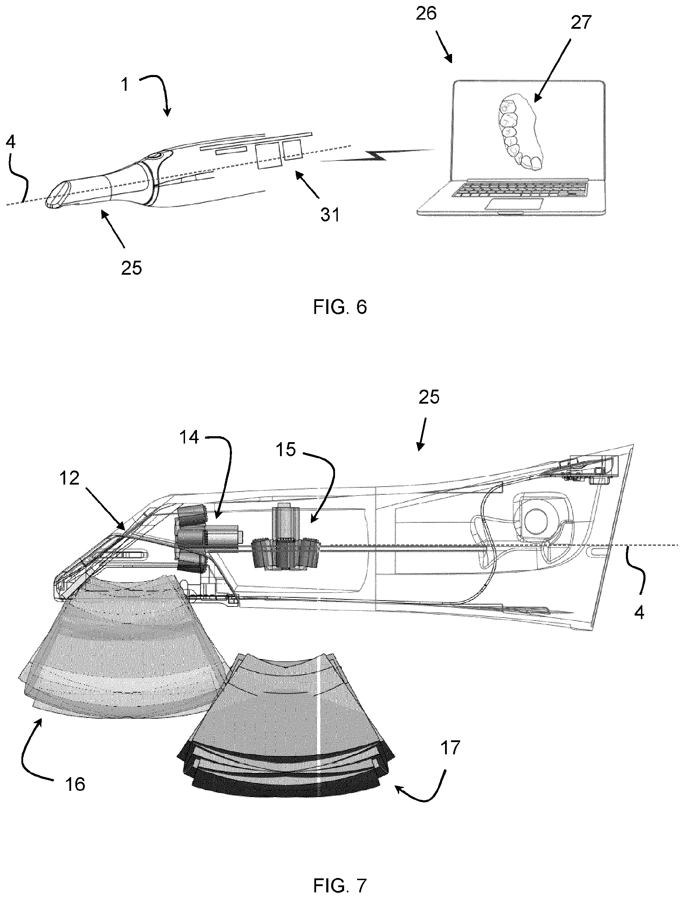

Accordingly, this embodiment of the scanning device features two scan units, each scan unit comprising a projector unit defining a projector optical axis, wherein the two scan units are oriented differently. This embodiment is illustrated in figure 7. In other words, one scan unit is configured as forward-looking and the other scan unit is configured as downward-looking. Only the first scan unit comprises a reflecting element to change the direction of the projected light, such that both of the projector units project light onto a surface of the dental object. An advantage of this embodiment is that two different working distances are achieved using similar optics / scan units. Thereby, effectively two different focus distances below the probe of the scanning device are achieved. In principle, if the two projector units have approximately similar focus distances when measured along the optical path, then if one of the optical paths is longer than the other (e.g. for the first scan unit), the result is that the scanning device has two different focus distances for the scan unit, when measured below a window/opening of each scan unit. An advantage hereof is that typically the user, such as the dentist, holds the scanning device in a slightly angled position when scanning the subject/patient. Thus, typically the most distal part of the scanning device / probe will be closer to the dental object than a part positioned closer towards the proximal end of the scanning device / probe. This embodiment accounts for such use of the scanning device, wherein the scanning device is held in a slightly elevated position.

In yet another embodiment, the intraoral scanning device comprises an elongated probe defining a longitudinal axis of the scanning device, a first scan unit comprising a first projector unit configured to project a light pattern onto a surface of the dental object, wherein the first projector unit defines a first projector optical axis; at least one camera comprising an image sensor for acquiring images, wherein the at least one camera defines a camera optical axis; and a reflecting element configured to reflect light from the first projector unit and/or reflect light from the surface of the dental object and onto the image sensor(s) of each camera. In this embodiment, the scanning device further comprises a second projector unit configured to project a light pattern onto a surface of the dental object, wherein the second projector unit defines a second projector optical axis; and at least one camera comprising an image sensor for acquiring images, wherein the at least one camera defines a camera optical axis, wherein each scan unit defines a field of view (FOV).

In yet another embodiment, the intraoral scanning device comprises an elongated probe defining a longitudinal axis of the scanning device; at least two scan units, each scan unit comprising: a projector unit configured to project a light pattern onto a surface of a dental object, wherein the projector unit defines a projector optical axis; at least one camera comprising an image sensor for acquiring images, wherein the at least one camera defines a camera optical axis; and a reflecting element configured to reflect light from the projector unit and/or reflect light from the surface of the dental object and onto the image sensor(s) of each camera; wherein each scan unit defines a field of view (FOV), and wherein the at least two scan units are located in the elongated probe and arranged to project the light pattern on the same side of said probe.

Accordingly, the scanning device disclosed herein provides a number of advantages compared to existing scanning devices.

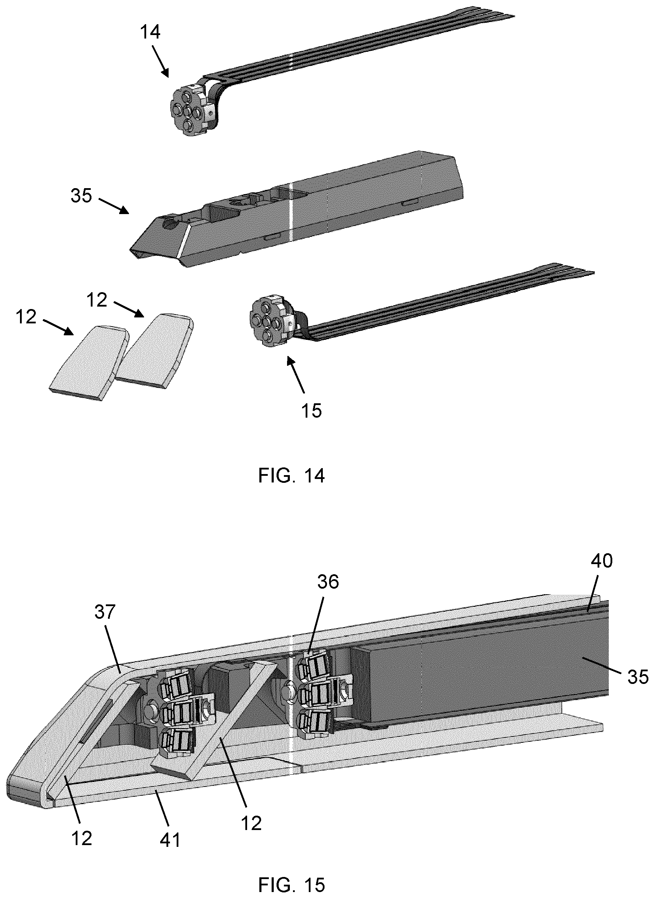

The present disclosure further relates to a method of assembling an intraoral scanning device as disclosed herein. The method is particularly relevant for assembling a scanning device, wherein the scanning device comprises a first scan unit and a second scan unit, and wherein the scanning device further comprises a reflecting element, such as a mirror, associated with each scan unit. In other words, each reflecting element is preferably positioned and mounted in the frame such that light projected from a given scan unit is incident on the reflecting element associated with said scan unit. In some embodiments, the method of assembling the scanning device disclosed herein, comprises the steps of: providing a frame for accommodating the scan units, wherein the frame comprises two or more openings for receiving the scan units; inserting a first scan unit into the frame through a first opening located in a first surface of the frame; and inserting a second scan unit into the frame through a second opening located in a second surface of the frame. The first and second surfaces may be opposite each other, such that one scan unit is inserted into the frame from above and the other scan unit inserted from below as illustrated in figure 14.

### Brief description of the drawings

Fig. 1 shows a side-view of a scan unit comprising a projector unit and four cameras.
Fig. 2 shows a perspective view of a scan unit comprising a projector unit and four cameras.
Fig. 3 shows a side-view of two scan units positioned in series along the longitudinal direction of the scanning device.
Fig. 4 shows a side-view of a scan unit comprising a projector unit, four cameras, and a reflecting element, such as a mirror.
Fig. 5A shows a scanning device comprising two scan units, such that the scanning device has an extended field of view along the longitudinal direction ('long FOV' configuration).
Fig. 5B shows the scanning device of fig. 5A seen from the distal end of the scanning device.
Fig. 5C shows a side-view of a scanning device comprising two scan units, such that the scanning device has an extended field of view ('double swipe' configuration).
Fig. 5D shows the scanning device of fig. 5C seen from the distal end of the scanning device.
Fig. 5E shows a side-view of a scanning device comprising two scan units, such that the scanning device has an extended field of view ('single swipe' configuration).
Fig. 5F shows the scanning device of fig. 5E seen from the distal end of the scanning device.
Fig. 6 shows a dental scanning system comprising an intraoral scanning device and a computer system, here a laptop.
Fig. 7 shows a side-view of a scanning device, in particular the tip of the scanning device, wherein the scanning device comprises two scan units in two different configurations.
Figs. 8A-8D show some examples of how the cameras and projector unit may be positioned and/or oriented when viewed along the projector optical axis.
Fig. 9 shows a perspective view of a scan unit comprising a projector unit and four cameras, wherein the scan unit further comprises a reflecting element, here shown as a mirror.
Fig. 10 shows an embodiment of a scanning device comprising two scan units, wherein the scanning device further comprises a wireless module configured to wirelessly transfer scan data, such as images and/or 3D data, to a computer system.
Fig. 11 shows an embodiment of a scanning device comprising one scan unit, wherein the scanning device is configured to transfer scan data using a wired connection.
Fig. 12 shows an embodiment of a scanning device comprising a first scan unit and a second scan unit, wherein each scan unit further comprises a reflecting element, here a mirror.
Fig. 13 shows the same embodiment as shown in fig. 12. In this visualization, a frame of the scanning device is shown. The frame is configured to accommodate the scan units.
Fig. 14 shows the same embodiment as shown in fig. 13; here shown as an exploded view. In this embodiment, the frame comprises a first opening for receiving the first scan unit, and a second opening for receiving the second scan unit.
Fig. 15 shows the same embodiment as shown in figures 12-14, except that in this figure both the frame, and an outer shell, as well as an optical window of the scanning device is visualized.
Fig. 16 shows a perspective view of an intraoral scanning device according to the present disclosure.
Fig. 17 shows a perspective view of the same embodiment shown in fig. 16; however, in this view, the replaceable part is shown detached from the scanning device.
Fig. 18 shows a perspective view of an intraoral scanning device according to the present disclosure.
Fig. 19 shows a cross-sectional view of an embodiment of an intraoral scanning device according to the present disclosure.
Fig. 20 shows a perspective view of an intraoral scanning device according to the present disclosure.
Fig. 21 shows an embodiment of a dental scanning system comprising an intraoral scanning device and a computer system, here exemplified as a laptop.
Fig. 22 shows three different embodiments of how to provide a chairside solution of a dental scanning system according to the present disclosure.

### Detailed description

### Three-dimensional object

The three-dimensional (3D) object may be a dental object. Examples of dental objects include any one or more of: tooth/teeth, gingiva, implant(s), dental restoration(s), dental prostheses, edentulous ridge(s), and/or combinations thereof. Alternatively, the dental object may be a gypsum model or a plastic model representing a subject's teeth. As an example, the three-dimensional (3D) object may comprise teeth and/or gingiva of a subject. The dental object may only be a part of the subject's teeth and/or oral cavity, since the entire set of teeth of the subject is not necessarily scanned during a scanning session. A scanning session may be understood herein as a period of time during which data (such as images) of the 3D object is obtained.

### Scanning device

The scanning device may be an intraoral scanning device for acquiring images within an intraoral cavity of a subject. The scanning device may employ a scanning principle such as triangulation-based scanning or stereo vision. In preferred embodiments, the scanning device employs a triangulation-based scanning principle. The scanning device comprises at least one projector unit and at least one camera. The scanning device may be a handheld scanning device, i.e. a device configured to be held with a human hand.

The scanning device comprises one or more scan units, such as at least two scan units, wherein each scan unit comprises a projector unit and one or more cameras. As an example, the scanning device may comprise one scan unit comprising one projector unit and at least two cameras. As another example, the scanning device may comprise one scan unit comprising one projector unit and four cameras. In yet another example, the scanning device may comprise at least two scan units, wherein each scan unit comprises a projector unit and two or more cameras. In yet another example, the scanning device may comprise at least two scan units, wherein each scan unit comprises a projector unit and four cameras.

Any of the scan units may further comprise a reflecting element. The reflecting element is preferably configured to reflect light from the projector unit(s) of the scan unit featuring the reflecting element and/or reflect light from the surface of the dental object and onto the image sensor(s) of each camera of the scan unit featuring the reflecting element. In preferred embodiments, the scanning device comprises an elongated probe, which defines a longitudinal axis of the scanning device.

### Projector unit

A projector unit may be understood herein as a device configured for generating an illumination pattern to be projected onto a surface, such as the surface of the three-dimensional object. In preferred embodiments, the projector unit(s) are configured to project a light pattern onto the surface of a dental object. Preferably, the projector units are configured to project the light pattern such that the light pattern is in focus at a predefined focus distance measured along the projector optical axis. In some embodiments, the predefined focus distance differs among the projector units of the scan units. In other embodiments, the focus distances of the projector units are largely similar along the optical axis of each scan unit; however, due to different positions and/or orientations of the scan units within the scanning device, the focus distances when measured a distance below the tip of the scanning device are different. Such an embodiment is illustrated in figure 7. In some embodiments, the projector unit is configured to project the light pattern such that the light pattern is defocused at the opening of the probe of the scanning device and/or at a surface of an optical window in said probe.

The projector unit(s) may be Digital Light Processing (DLP) projectors using a micro mirror array for generating a time varying pattern, or a diffractive optical element (DOE), or front-lit reflective mask projectors, or micro-LED projectors, or Liquid crystal on silicon (LCoS) projectors or back-lit mask projectors, wherein a light source is placed behind a mask having a spatial pattern, whereby the light projected on the surface of the dental object is patterned. The pattern may be dynamic, i.e. such that the pattern changes over time, or the pattern may be static in time, i.e. such that the pattern remains the same over time. The projector unit(s) may comprise one or more collimation lenses for collimating the light from the light source, said collimation lens(es) being placed between the light source and the mask. The collimation lens(es) may comprise one or more Fresnel lenses. The projector unit(s) may further comprise one or more focus lenses configured for focusing the light at a predefined focus distance or working distance.

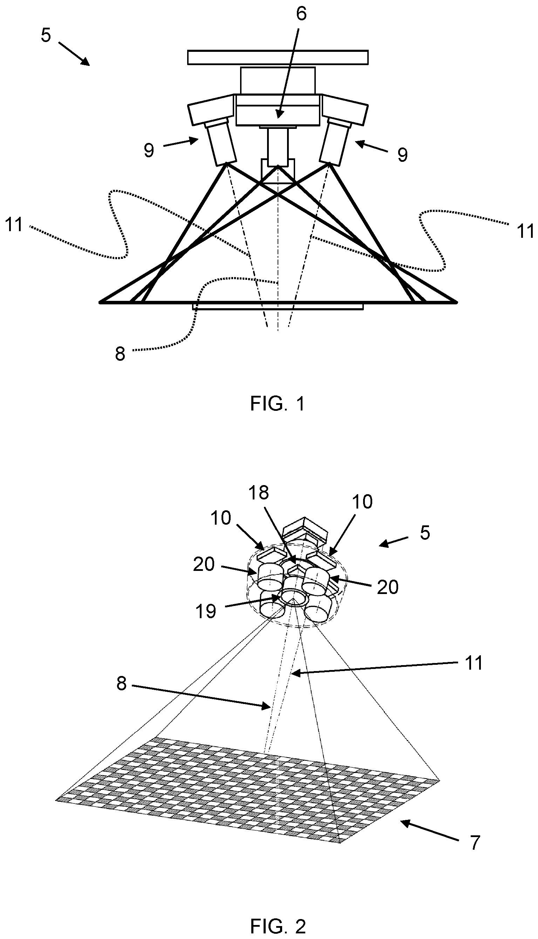

In preferred embodiments, the projector unit(s) of the scanning device comprise at least one light source and an optical mask having a spatial pattern. Hence, each projector unit may comprise an optical mask configured to define the light pattern. The optical mask may be similar to a small photomask, such as a chrome-on-glass photomask. As an example, the optical mask may define a polygonal pattern comprising a plurality of polygons, such as a checkerboard pattern, as illustrated in figure 2. In other embodiments, the projector unit(s) comprises at least one light source and a pattern generating element, such as a diffractive optical element (DOE), for defining the spatial pattern. The projector unit(s) may further comprise one or more lenses such as collimation lenses or projection lenses.

Preferably, the projector unit(s) are configured to generate a predefined static pattern, which may be projected onto a surface. A first advantage of using a static light pattern is that no moving parts are necessary in the projector unit for projecting the static light pattern. A further advantage is that the projector unit can be made quite small and thereby for example be integrated in the tip of the intraoral scanning device thereby reducing the form factor of the tip and/or the overall size of the intraoral scanning device. A further advantage of projecting a static pattern is that it allows the capture of all image data simultaneously, thus preventing warping due to movement between the scanning device and the object.

Alternatively, the projector unit(s) may be configured to generate a dynamic pattern, which changes in time. Each of the projector units is associated with their own projector plane, which is determined by the projector optics. As an example, if the projector unit is a back-lit mask projector, the projector plane may be understood as the plane wherein the mask is contained. The projector plane comprises a plurality of pattern features of the projected pattern.

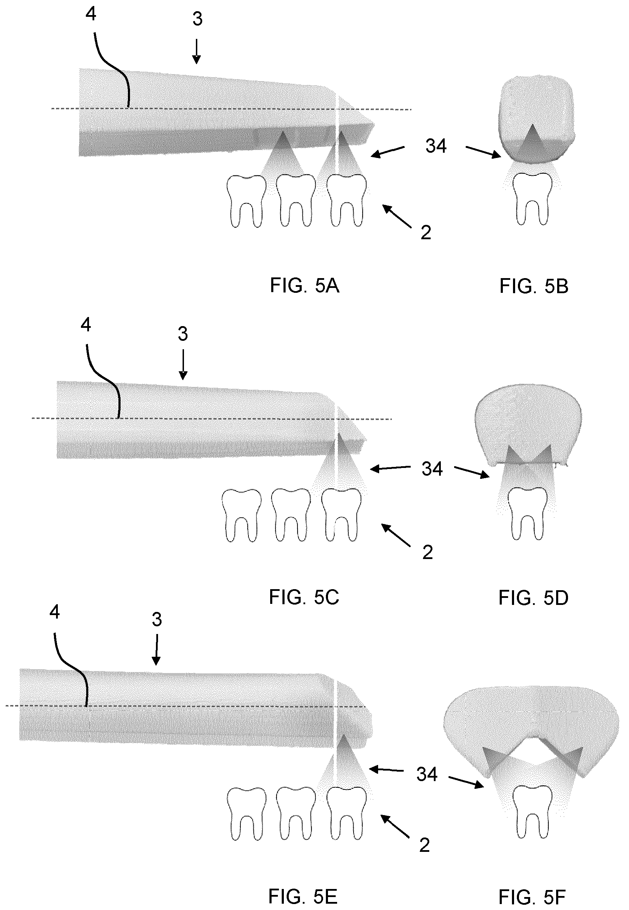

Preferably, each projector unit defines a projector optical axis. An optical axis may be understood as a line along which there is some degree of rotational symmetry in an optical system such as a camera lens or a projector unit. In some embodiments, the projector optical axis of one or more of the projector units is substantially parallel with the longitudinal axis of the scanning device. In some embodiments, the projector optical axis of each projector unit is substantially parallel with the longitudinal axis of the scanning device. In other embodiments, a first projector optical axis of a first scan unit defines an angle, such as at least 45° or at least 75°, with a second projector optical axis of a second scan unit. In other embodiments, the projector optical axis of a projector unit of a first scan unit is substantially parallel with the longitudinal axis of the scanning device, and the projector optical axis of a projector unit of a second scan unit is substantially orthogonal to the longitudinal axis of the scanning device. Thus, in some embodiments, the first and second projector optical axes define an angle of at least 85°, such as approximately 90°. In some embodiments, this angle is measured in a plane parallel to the longitudinal axis of the scanning device, cf. fig. 6. In other embodiments, this angle is measured in a plane orthogonal to the longitudinal axis of the scanning device, cf. fig. 5D and fig. 5F.

The projector unit may be configured for sequentially turning the light source on and off at a predetermined frequency, wherein the light source is on for a predetermined time period. As an example, the time period may be between 3 milliseconds (ms) and 10 milliseconds (ms), such as between 4 ms and 8 ms. Through experiments, the inventors have found that a time period of between 4 ms and 8 ms is particularly suitable for ensuring a good compromise between exposure of the pixels and minimizing blurring of the images due to relative movement between the scanning device and the scanned object. The predetermined frequency for turning the light source on and off may be between 25 Hz and 35 Hz, such as approximately 30 Hz. In some embodiments, all pixels of the image sensor(s) are constantly exposed. In that case, the effective exposure is controlled by the flash period of the light source. In some embodiments, the exposure time of the image sensor is below 15 milliseconds (ms), such as below 10 ms, such as between 4 to 8 ms. These indicated exposure times preferably correspond to the time period of the flash of the light source of the projector unit as described above. An advantage of configuring the light source to flash during a time period as indicated above, is that blurring due to relative movement between the scanning device and the object being scanned is minimized. This kind of blurring is also referred to as motion blur. The light source of the projector unit may be configured to generate unpolarized light, such as unpolarized white light. This has the advantage of providing a better signal-to-noise ratio, and consequently a lower noise level in the generated 3D representation. This may be due to the fact that more specular light is reflected from the surface in case of polarized light.

The projector unit may be configured to project a plurality of projector rays, which are projected onto a surface of the dental object. Solving the correspondence problem may include the steps of determining image features in the images within a set of images, and further associate said image features with a specific projector ray. In preferred embodiments, the correspondence problem is solved jointly for groups of projector rays, as opposed to e.g. solving the correspondence problem projector ray by projector ray. The group of projector rays considered may form a connected subset within the pattern. The inventors have found that by solving the correspondence problem jointly for groups or collections of projector rays, a particular reliable and robust solution can be obtained, consequently leading to a more accurate 3D representation. Subsequently, the depth of each projector ray may be computed, whereby a 3D representation of the scanned object may be generated.

The projector unit may comprise an aperture having a predetermined size such that it provides a pupil diameter of between 0.2 mm to 0.7 mm, such as between 0.3 mm to 0.6 mm. The pupil diameter may be understood as the entrance pupil or the exit pupil. In some cases, the entrance pupil and the exit pupil are of similar size. In experiments performed by the inventors, a pupil diameter of between 0.2 mm to 0.7 mm was found to be particularly useful because it provided a projected pattern in particularly good focus in a large focus range, e.g. a focus range of between 16 mm and 22 mm. In particular, a pupil diameter between from about 0.3 mm to about 0.5 mm was found to provide a good compromise between the imaging resolution, e.g. the resolution of the pattern, and the depth of focus, i.e. the focus range. Depth of focus may in some cases be understood as the maximum range where the object appears to be in acceptable focus, e.g. within a given predetermined tolerance. A further advantage of a pupil diameter of between 0.2 mm to 0.7 mm is that it provides a good ability to collect light, which in turn means that the pixels on the image sensor(s) can be a exposed for a shorter time period. A shorter exposure is advantageous to minimize motion blur.

### Light source

The light source(s) may be configured to generate light of a single wavelength or a combination or range of wavelengths (mono- or polychromatic). The combination of wavelengths may be produced by a light source configured to produce light comprising different wavelengths, or light having a range of wavelengths, such as white light. In preferred embodiments, each projector unit comprises a light source for generating white light. The white light source may be used in combination with the optical mask to form a back-lit mask projector unit. An advantage of utilizing white light is that it enables the scanning device to acquire data relating to the surface geometry and to the surface color simultaneously. Consequently, the same set of images can be used to provide both the 3D geometry of the object and color of the object. Hence, there is no need for an alignment of data relating to the recorded surface geometry and data relating to the recorded surface color in order to generate a digital 3D representation of the object expressing both color and geometry of the object. Alternatively, the projector unit(s) may comprise multiple light sources such as LEDs individually producing light of different wavelengths (such as red, green, and blue) that may be combined to form light comprising different wavelengths. Thus, the light produced by the light source(s) may be defined by a wavelength defining a specific color, or a range of different wavelengths defining a combination of colors such as white light. In some embodiments, the projector unit comprises a laser, such as a blue or green laser diode for generating blue or green light, respectively. An advantage hereof is that a more efficient projector unit can be realized, which enables a faster exposure compared to utilizing e.g. a white light diode.

In some embodiments, the scanning device comprises a light source configured for exciting fluorescent material to obtain fluorescence data from the dental object such as from teeth. Such a light source may be configured to produce a narrow range of wavelengths, such as in the range of between 380 nm and 495 nm, such as between 395 nm to 435 nm. In some embodiments, the scanning device comprises an infrared light source, which is configured to generate wavelengths in the infrared range, such as between 700 nm and 1.5 µm. The infrared light may be capable of penetrating dental tissue to obtain data of an internal region of the tooth or teeth. In some embodiments, the scanning device comprises a light source selected from the group of: Infrared (IR) light source, near-infrared (NIR) light source, blue light source, violet light source, and/or combinations thereof. The scanning device may further comprise one or more NIR light sources, such as a plurality of NIR light sources, for emitting near-infrared light having a wavelength range of about 750 nm to about 1.4 µm. Light in the infrared range may be used for diagnostic purposes, e.g. for detecting regions of caries inside an oral cavity. In particular, infrared (IR) light, such as NIR light, penetrates the enamel of teeth relatively easily and is reflected by porous areas (e.g. carious lesions). Thus, carious regions appear light(er) in IR images captured by the scanning device.

### Light pattern

The terms 'light pattern', 'illumination pattern', 'spatial pattern', and 'pattern' are used herein interchangeably. The pattern is preferably defined by a mask having a spatial pattern. The spatial pattern may be a polygonal pattern comprising a plurality of polygons. The polygons may be selected from the group of: triangles, rectangles, squares, pentagons, hexagons, and/or combinations thereof. Other polygons can also be envisioned. In general, the polygons are composed of edges and corners. In preferred embodiments, the polygons are repeated in the pattern in a predefined manner. As an example, the pattern may comprise a plurality of repeating units, wherein each repeating unit comprises a predefined number of polygons, wherein the repeating units are repeated throughout the pattern. Alternatively, the pattern may comprise a predefined arrangement comprising any of stripes, squares, dots, triangles, rectangles, and/or combinations thereof. In some embodiments, the pattern is non-coded, such that no part of the pattern is unique. In other embodiments, the pattern is a pseudo random square dot pattern.

In preferred embodiments, the generated illumination pattern is a polygonal pattern, such as a checkerboard pattern comprising a plurality of checkers. Similar to a common checkerboard, the checkers in the pattern have alternating dark and bright areas corresponding to areas of low light intensity (dark) and areas of high(er) light intensity (bright). Such a pattern is exemplified in fig. 2. In some cases, the checkers of the checkerboard pattern may be referred to herein as white (bright) and black (dark) checkers. In some embodiments the light pattern is a checkerboard pattern comprising alternating checkers (also referred to as squares) of dark and bright. As an example, the checkerboard pattern may comprise at least 100 × 100 squares of alternating dark and bright squares, preferably at least 120 × 120 squares, even more preferably at least 140 × 140 squares. In some embodiments, each checker in the checkerboard pattern has a length of between 100 µm to 200 µm. This may in some cases correspond to a pixel size of between 4-16 pixels, such as between 8-12 pixels, when the pattern is imaged on the image sensor(s). Through experiments the inventors have realized that at least 4 resolvable pixels per checker period is sufficient to get a reasonable contrast and well-defined edges of the projected checkerboard pattern.

The pattern preferably comprises a plurality of pattern features. In some embodiments, the light pattern comprises at least 1000 pattern features, such as at least 3000 pattern features, or at least 5000 pattern features, or even at least 10000 pattern features. A system and method for projecting a high-density pattern is further described in PCT/EP2023/058521 by the same applicant. When projecting a pattern comprising such pattern features onto a surface of the 3D object, the acquired images of the object will similarly comprise a plurality of image features corresponding to the pattern features. A pattern/image feature may be understood as an individual well-defined location in the pattern/image. Examples of image/pattern features include corners, edges, vertices, points, transitions, dots, stripes, etc. In preferred embodiments, the image/pattern features comprise the corners of checkers in a checkerboard pattern. In other embodiments, the image/pattern features comprise corners in a polygon pattern such as a triangular pattern. The light pattern may be a structured light pattern having a distribution of discrete unconnected spots of light, wherein said spots of light corresponds to pattern features.

### Camera

A camera may be understood herein as a device for capturing an image of an object. Each camera comprises an image sensor for generating an image based on incoming light e.g. received from the illuminated 3D object. Each camera may further comprise one or more lenses for focusing light. As an example, the image sensor may be an electronic image sensor such as a charge-coupled device (CCD) or an active-pixel sensor (CMOS sensor). The image sensor may comprise an array of pixels, such as a two-dimensional array of pixels. In some embodiments, the array of pixels is quadratic. In some embodiments, the array of pixels comprises at least 800 x 800 pixels, such as at least 1200 x 1200 pixels. This ensures a good compromise between size of the image sensor and image resolution, which directly influences the 3D resolution. In some cases, the projected pattern is a checkerboard pattern, wherein the side of each checker is between 8 pixels to 12 pixels. This has the advantage of improving the determination of the position of the image features, and thereby improving the 3D accuracy, since said position of image features directly influences the determination of 3D points. Furthermore, in some cases, generating color requires multiple pixels, e.g. in the case of a Bayer filter, which means that the color resolution and/or the 3D resolution may differ from the resolution of the image sensor(s).

The image sensor(s) may have a frame rate of at least 30 frames per second, such as at least 60 frames per second, or even at least 75 frames per second. Thus, in some applications, the camera units may capture images, or sets of images, at a frame rate of at least 30 frames per second, e.g. at a frame of at least 60 frames per second, e.g. at least 75 frames per second. The number of 3D frames generated by the scanner per second may correspond to, or be less than, the above-indicated frame rates of the image sensors.

The image sensor may be a global shutter sensor or a rolling shutter sensor. An advantage of utilizing a rolling shutter sensor is that the sensor can be made smaller for a given pixel array size compared to e.g. a global shutter sensor, which typically comprises more electronics per pixel leading to a sensor having a larger area or volume footprint, thus taking up more space. Thus, a rolling shutter sensor is advantageous for applications with restricted space, such as for intraoral scanning devices, in particular for realizing a compact intraoral scanning device. The rolling shutter sensor is particular advantageous when used in combination with the flashing sequence of the light source as described above, since motion blur is limited due to the short flash of e.g. 4 ms to 8 ms, and further since the flashing minimizes or completely avoids rolling shutter distortion.

Each image sensor may define an image plane, which is the plane that contains the object's projected image. Each image obtained by the image sensor(s) may comprise a plurality of image features, wherein each image feature originates from a pattern feature of the projected pattern. In some embodiments, the scanning device comprises one or more processors configured to determine the image features, e.g. using a trained neural network. In some cases, the neural network is executed by a neural processing unit (NPU). Training of the neural network is further described in PCT/EP2022/086763 by the same applicant.

In some embodiments, at least one of the camera(s) in at least one of the scan units is a light field camera. In other embodiments, each scan unit comprises at least one light field camera. Preferably, each camera defines a camera optical axis. In some embodiments, there is a color filter array, such as a Bayer filter, arranged on the image sensor(s). The color filter array may comprise a mosaic of tiny color filters placed over the pixel sensors of each image sensor to capture color information. The image sensor(s) may be color image sensors or alternatively monochrome image sensors.

In accordance with some embodiments, the projector optical axis and the camera optical axis of at least one camera define a camera-projector angle of approximately 5 to 15 degrees, preferably 5 to 10 degrees, even more preferably 8 to 10 degrees. In experiments performed by the inventors, a camera-projector angle of between 5 to 10 degrees was found to be particularly useful because angles within this range increased the overlap between cameras and the projector unit, which consequently removed the need to overdesign field of view (FOV) optical requirements of the scanning device. The term 'overdesign' may be understood as to design in a manner that is excessively complex or that exceeds usual standards. A further advantage is that a camera-projector angle of between 5 to 10 degrees significantly improved the ability to scan hard-to-reach areas like cavities inside the oral cavity of a subject, e.g. inside the mouth of a patient. Thus, angles within this range are particularly suitable for dental scanning applications using an intraoral scanning device. It was further found that a camera-projector angle of less than 5 degrees introduced more noise in the obtained data, in particular with respect to depth determination, which made it more difficult to determine 3D points by triangulation. An optimal angle was found between 7 to 10 degrees, such as between 8 to 10 degrees. In embodiments employing multiple scan units, e.g. two or more scan units, wherein each scan unit comprises at least one pair of camera and projector unit, the camera-projector angle is preferably substantially the same for both scan units. In some embodiments, the cameras are defocused at the opening of the probe of the scanning device and/or at the surface of an optical window in said probe. In preferred embodiments of the scanning device, the cameras and projector unit(s) of a given scan unit are focused at the same distance. The cameras may be configured to acquire a set of images, wherein a correspondence problem is solved within said set of images based on triangulation. The images within the set of images may be acquired by separate cameras of a given scan unit of the scanning device. The images within the set of images are preferably acquired simultaneously, i.e. at the same moment in time, wherein each camera contribute with one image to the set of images. An advantage hereof, is that the light-budget is improved; thus, less power is consumed by the light source and the projector unit. The images within the set of images preferably captures substantially the same region of the dental object. The images may comprise a plurality of image features corresponding to pattern features in a structured pattern projected on the surface of the dental object. The correspondence problem may refer to the task of associating each image feature with a projector ray emanating from the projector unit. In other words, the problem can also be stated as the task of associating points in the images with points in the projector plane of the projector unit. A system and method for solving the correspondence problem is further described in PCT/EP2022/086763 by the same applicant.

### Scan unit

A scan unit may be understood herein as a unit comprising at least one projector unit and one or more cameras. In some embodiments, each scan unit comprises at least two cameras having at least partly overlapping fields of view along different camera optical axes. Preferably, each scan unit comprises at least four cameras having at least partly overlapping fields of view along different camera optical axes. An advantage of having overlapping fields of view of the cameras is an improved accuracy due to a reduced amount of image stitching errors. A further advantage of utilizing multiple cameras, such as two or more cameras, is that the reliability of the determination of 3D points is improved, whereby the accuracy of the generated digital 3D representation is improved. A scan unit may further comprise one or more lenses such as collimation lenses or projection lenses.

Figure 4 shows an embodiment of a scan unit, viewed in a cross-sectional view from the side, wherein the scan unit comprises a projector unit, at least two cameras, and a reflecting element, here exemplified as a mirror. The figure defines a number of dimensions of the scan unit.

In fig. 4, 'a' denotes the baseline, which may be understood as the center-to-center distance between a given camera and the projector unit. As an example, the baseline may be less than 5 mm, such as less than 4 mm, such as approximately 3 mm. Accordingly, in some embodiments, for a given scan unit, the center-to-center distance between the projector unit and a given camera of that scan unit is between 2 to 5 mm, preferably between 3 to 4 mm. In these examples, the working distance of the scan unit is approximately 24 mm.

In fig. 4, 'b' denotes the distance from the projector optical axis to the outer edge of the scan unit as illustrated in the figure. Preferably, the top of the scan unit does not extend beyond a horizontal line drawn from the top of the reflecting element. In other words, in preferred embodiments, the value of 'b' is chosen such that the scan unit does not extend beyond the top of the reflecting element.

In fig. 4, 'c' denotes the distance between the projector unit and the reflecting element, said distance measured along the projector optical axis. Preferably, this distance is as small as possible in order to minimize the dimensions of the reflecting element. In some embodiments, the distance 'c' is chosen such that a bottom corner and/or edge of the scan unit does not cut off light rays projected by the projector unit and/or does not cut off rays reflected from the scanned object. This corner is highlighted with a circle in figure 4. In some embodiments, the scanning device comprises a first scan unit and a second scan unit, wherein the first scan unit is positioned as close to the second scan unit as possible without cutting off rays that have been reflected by the reflecting element of the first scan unit. In some embodiment, the second scan unit further comprises a reflecting element configured to reflect light from the second projector unit and/or reflect light from the surface of the dental object and onto the image sensor(s) of each camera of the second scan unit.

In fig. 4, 'd' denotes the length of the scan unit. Typically, this length is measured along the projector optical axis. It is preferred that the length is relatively short, such that a second scan unit including mirror can be positioned 'behind' the first scan unit, as illustrated in fig. 3. This allows the total field of view of the scanning device to be increased compared to a scanning device with only one scan unit, while simultaneously keeping the tip height of the scanning device low. In particular, a short distance 'd' allows for positioning scan units close together, thus allowing the FOV of the scanning device to be spatially continuous, i.e. such that the FOV of neighboring scan units are aligned to form a combined FOV, ideally without non-covered areas in between the individual FOVs of the scan units.

In fig. 4, 'e' denotes the camera-projector angle, which is the angle between a given camera optical axis and the projector optical axis. In preferred embodiments, this angle is between 5-25 degrees, such as between 5-15, such as between 8-10 degrees.

In preferred embodiments, the scanning device comprises a plurality of scan units positioned along the longitudinal axis of the scanning device. Figure 3 shows an embodiment, wherein two scan units are positioned in series along the longitudinal axis of the scanning device. For simplicity, only the scan units including the reflecting elements are shown in the figure and not the entirety of the scanning device and its components. Preferably, the scan units are positioned at a distal end of the elongated probe, e.g. in the tip of the scanning device.

A scan unit may have a predefined field of view (FOV) and/or a predefined angular field of view (AFOV). The field of view (FOV) of a scan unit / scanning device may be understood as the extent of the observable world that is seen at any given moment by the scan unit / scanning device. The FOV may be reported as an area measure, e.g. an area at a given distance from the scan unit or at a given distance below the probe of the scanning device. Preferably, the FOV of each scan unit is at least 300 mm², preferably at least 400 mm², preferably at least 500 mm², preferably at least 550 mm², wherein the area is measured for a given predefined focus distance or working distance. As an example, the field of view of the scanning device may be at least 20 x 20 mm², such as at least 23 x 23 mm², at a working distance of between 18 mm to 36 mm, such as approximately 20 mm to 24 mm. For some applications, e.g. for dental scanning applications, it is preferred that the scanning device has a working distance of between 10 mm and 100 mm. In experiments performed by the inventors, it has been found that a working distance of the projector unit of between 10 mm and 70 mm, such as between 15 mm and 50 mm, is particularly useful, since the optics, e.g. the scan unit(s), take up less space inside the scanning device, and also since it is desired to be able to scan objects very close to the scanning device. Since the scan unit(s) then take up less space inside the scanning device, it also allows for multiple scan units to be placed in succession inside the scanning device. In preferred embodiments, the scanning device is able to project a pattern in focus at the exit of the tip of the scanning device, e.g. at the optical window of the scanning device or at an opening in the surface of the scanning device. The working distance may be understood as the object to lens distance where the image is at its sharpest focus. The working distance may also, or alternatively, be understood as the distance from the object to a front lens, e.g. a front lens of the projector unit. The front lens may be the one or more focus lenses of the projector unit.

In preferred embodiments the elongated probe comprises one or more openings and/or one or more optical windows at a distal end of the elongated probe. Preferably, there is an opening and/or an optical window in the elongated probe, wherein the opening/window is associated with each scan unit, such that each scan unit is configured to project the light pattern through said opening or optical window. Accordingly, the elongated probe may comprise an opening and/or an optical window for each scan unit of the scanning device. The optical window may be made in a polymer material such as poly(methyl methacrylate) (PMMA) or in a ceramic or glass material such as Sapphire glass. The optical window may be made in a transparent, crystalline, ceramic material, such as aluminium oxide (Al₂O₃). An advantage hereof is that the optical window is strong and resistant to scratches. Alternatively, the optical window may be made of a mineral glass. An advantage hereof is that it offers a good compromise between transparency, durability and cost. Preferably, the predefined distance mentioned in relation to the FOV of a given scan unit is measured below said opening/window. In preferred embodiments, the FOV of at least one scan unit is at least 500 mm², such as approximately 23 × 23 mm², in a distance approximately 0-15 mm, such as 4-7 mm below said opening/window. This may correspond to a working distance of between 15 mm and 50 mm, such as between 15 mm and 36 mm.

The angular field of view (AFOV) of a scan unit / the scanning device is correlated to the FOV. However, herein the AFOV is expressed as angles and the FOV is expressed as an area. In accordance with some embodiments, each scan unit defines an angular field of view (AFOV) of between 50° to 80°, preferably between 60° to 75°. In accordance with some embodiments, the projector optical axis and the camera optical axis of at least one camera define a camera-projector angle of approximately 5 to 25 degrees, preferably 5 to 15 degrees, preferably 5 to 10 degrees, even more preferably 8 to 10 degrees. Preferably, the scan unit(s) are rotationally symmetric, such that each camera optical axis defines an approximately similar camera-projector angle with the projector unit of the scan unit.

Through experiments performed by the inventors, it has been determined a numerical aperture of between 0.0035 and 0.015 of the projector unit(s) and/or camera(s) enables the ability to resolve very fine details of between 50-200 µm in size in a focus range between 10 mm and 50 mm, such as between 12 mm and 36 mm. In some applications, the scan unit is configured to have a working distance of between 15 mm and 50 mm, e.g. the working distance of the projector unit(s) and/or the camera(s). In some cases, the working distance can be longer than 50 mm, e.g. in case the scanning device comprises a mirror arranged in the distal end of the scanning device. Conversely, the working distance may in some cases be shorter than 15 mm if the scan unit is provided without a mirror in the scanning device. A numerical aperture of between 0.0035 and 0.015 may correspond to apertures providing a pupil diameter of between 0.2 mm and 0.7 mm. Accordingly, a technical effect of this range of numerical aperture is that it provides the ability to project a high-density pattern, wherein the pattern is in focus in a relatively wide focus range in close proximity to the scanning device, and wherein the blurring of the pattern features is below a given tolerance. Consequently, a more accurate 3D representation may be generated since the position of the 3D points can be determined more accurately, i.e. with less uncertainty, and also since the smaller features allows for more features to be present in the pattern, thereby leading to a 3D representation comprising more 3D points.

In embodiments, wherein the scanning device comprises at least two scan units, the scanning device has a FOV, which is larger than the FOV of each scan unit. As an example, if the scanning device comprises two scan units, each scan unit having a predefined FOV, then the scanning device will have twice as large a FOV as each scan unit, provided there is no overlap in FOV between the scan units. In some cases, there may be a small overlap in FOV, but in general the scanning device will have a larger field of view, or in other words an extended field of view, compared to one scan unit in isolation. Accordingly, the FOV of the scanning device can be increased/scaled by the number of scan units in the scanning device. In some embodiments, the scanning device comprises at least three scan units. Accordingly, the scanning device may further comprise a third scan unit comprising a third projector unit, said projector unit preferably configured to project the light pattern at a predefined third focus distance.

A scan unit may further comprise at least one reflecting element, such as a mirror or a prism, wherein said reflecting element is configured to alter the direction of the light projected by the projector unit of the scan unit. In general, a scan unit can be oriented in many different ways, wherein the orientation is defined according to the projector optical axis of the scan unit. As an example, a scan unit may be oriented such that the projector optical axis of the scan unit is substantially parallel with the longitudinal axis of the scanning device. Additionally, a scan unit may be oriented such that the light from the projector unit is projected in a forward direction, i.e. towards the distal end of the scanning device. In case, a scan unit is oriented in this way, the scan unit is also referred to as being in a forward-looking configuration. In some embodiments, each scan unit comprises a reflecting element. Such an embodiment is illustrated in figure 3. In other embodiments, at least one scan unit comprises a reflecting element.

As another example, a scan unit may be oriented such that the projector optical axis of the scan unit is angled with respect to the longitudinal axis of the scanning device or with respect to a projector optical axis of another scan unit. In some embodiments, at least one of the projector optical axes is substantially orthogonal to the longitudinal axis of the scanning device. Additionally, such a scan unit may be oriented such that the light from the projector unit is projected in a downward direction, i.e. directly towards the dental object to be scanned / being scanned. In case, a scan unit is oriented in this way, the scan unit is also referred to as being in a downward-looking configuration. If the scanning device comprises a plurality of scan units, such as at least two scan units, the scan units may be in different configurations, e.g. a first scan unit in the forward-looking configuration and a second scan unit in the downward-looking configuration. Such an embodiment is illustrated in fig. 7. In this embodiment, the first projector optical axis is substantially parallel to the longitudinal axis of the scanning device, and the second projector optical axis is substantially orthogonal to the longitudinal axis of the scanning device. In other words, a first projector optical axis of a first projector unit of the first scan unit is angled with respect to a second projector optical axis of a second projector unit of the second scan unit. This angle is preferably at least 45°, such as at least 75°, even more preferably at least 85°, such as approximately 90°.

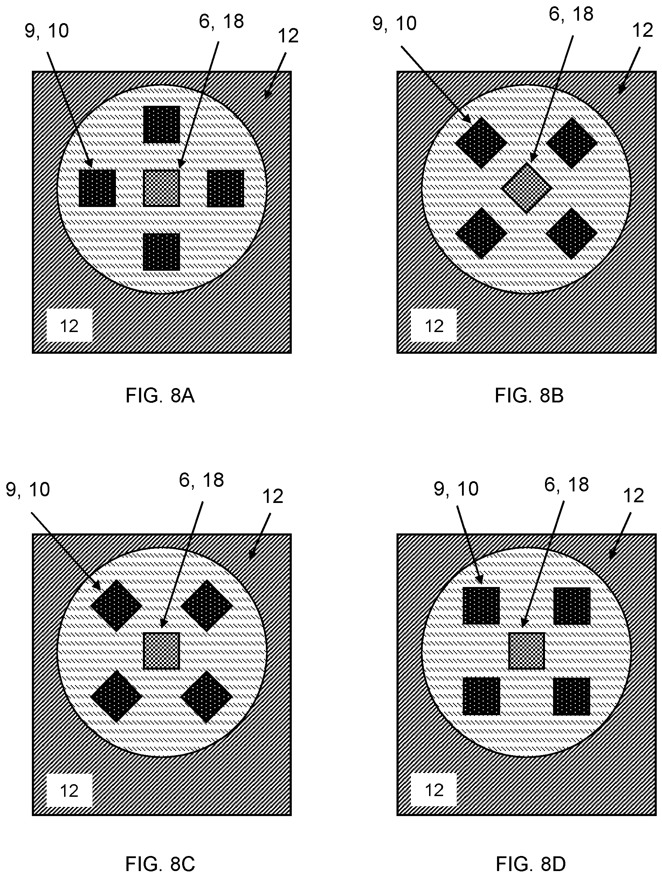

In some embodiments, each scan unit comprises one projector unit and a plurality of cameras, wherein, for each scan unit, the cameras are positioned substantially in the same plane. The cameras and projector unit of each scan unit may be positioned and/or oriented in said plane in a variety of ways. Figures 8A-8D display some examples of how the cameras and projector unit may be positioned/oriented in said plane. It should be noted that the plane represents a cross-section through the scan unit. Hence, in reality the components are not contained entirely in the same plane, since the components have a physical dimension extending outside this plane / cross-section.

In some embodiments, each scan unit comprises one projector unit and a plurality of cameras, such as four cameras, wherein the cameras are arranged on two axes to form a cross in a plane, wherein the projector unit is located in the center of the cross when viewed along the projector optical axis. This embodiment is illustrated in fig. 8A. In this embodiment, a first camera is located above the projector unit in the plane, a second camera is located below the projector in the plane, a third camera is located to the left of the projector in the plane, and a fourth camera is located to the right of the projector in the plane. Preferably, the two axes of the cross are substantially parallel with at least two edges of the reflecting element.

In particular, in the embodiment illustrated in figures 8A-8D, the image sensors, optical masks, and reflecting elements, each comprises four edges, said edges forming a rectangle. Some of the corners of the rectangle(s) may be rounded. Furthermore, in this embodiment, for each scan unit, one or more edges of the image sensor, optical mask, and reflecting element are substantially aligned, such that corresponding edges are substantially parallel.

As seen from fig. 8A, the edges of the image sensors are substantially parallel with the edges of the reflecting element, when viewed in this cross-section. The inventors have found that this particular arrangement has some benefits over other arrangements of the cameras and projector unit in the plane with respect to the reflecting element. One advantage is a good tracking uniformity, i.e. a more uniform tracking performance across each image sensor. Tracking may be understood as the task of solving the correspondence problem, i.e. the problem of ascertaining which parts of one image correspond to which parts of another image. In particular, the arrangement of the image sensors and optical mask in fig. 8A ensures a more uniform depth resolution across a given image sensor. This is due to the fact that the epipolar lines on a given image sensor are associated with a greater range in depth, since the epipolar lines are substantially aligned with the edges of a given image sensor and the optical mask. In other words, ideally all areas of the image sensor(s) cover similar depth ranges. Thus, the image sensor(s) of fig. 8A capture more depth information, especially in the corners of the image sensor(s), compared to e.g. the arrangement shown in fig. 8D. In the arrangement of fig. 8D, the epipolar lines are diagonal on the image sensors, which means that some corners of a given image sensor will capture less depth information compared to e.g. the center of the image sensor. Thus, for the arrangement in fig. 8D, the depth resolution is not as uniform across the image sensor(s) as for the arrangement in fig. 8A.

Another advantage of the arrangement shown in fig. 8A, is that it allows the dimensions of the reflecting element(s) to be kept at a minimum without cutting off rays. Consequently, the tip height of the scanning device can be made rather small, since the reflecting element affects the minimum size of the tip.

The embodiment illustrated in fig. 8B is largely similar to the embodiment of fig. 8A, however, in this embodiment, the cross is rotated 45 degrees with respect to the edges/sides of the reflecting element. In other words, the diagonals of the optical mask / image sensors are aligned with a vertical axis of the reflecting element. Because the angular field of view increases along the mask/sensor diagonals, the height of the reflecting element (here a mirror) must be increased to avoid cutting off rays. The mirror height corresponds to the height of the mirror when viewed from the side, as shown in figure 4. In some embodiments, the height of the mirror as seen along the projector optical axis (or from the side as shown in fig. 4) is between about 13 mm to about 20 mm. An advantage hereof is that the tip height of the scanning device is kept at a minimum, in particular for working distances above 15 mm. Thus, the mirror allows for part of the optical path to be folded or redirected inside the scanning device, such that the scanning device may accommodate an optical system, e.g. a scan unit, having a working distance longer than the intended height of the probe or tip of the scanning device. Consequently, the tip or probe can be made smaller, in particular the height of the probe, such that it may easily enter e.g. an oral cavity of a patient. A smaller probe also more easily captures data in the back of the mouth of the patient.

The scan unit may comprise a fixation unit configured for receiving and mounting the projector unit and the camera units. The fixation unit is preferably further configured such that each camera optical axis forms a predefined angle with the projector axis when mounted in the fixation unit. In preferred embodiments, the fixation unit is configured to accommodate at least one projector unit and two or more camera units, such as four camera units. The fixation unit may be rigid and made in one piece. An advantage hereof is that the geometric relationship between the projector unit and the camera unit(s) is fixed. In some embodiments, the fixation unit is made from stainless steel, which has the advantage of being a good thermal conductor, thus having a good ability to remove heat from e.g. the projector unit.

### Reflecting element

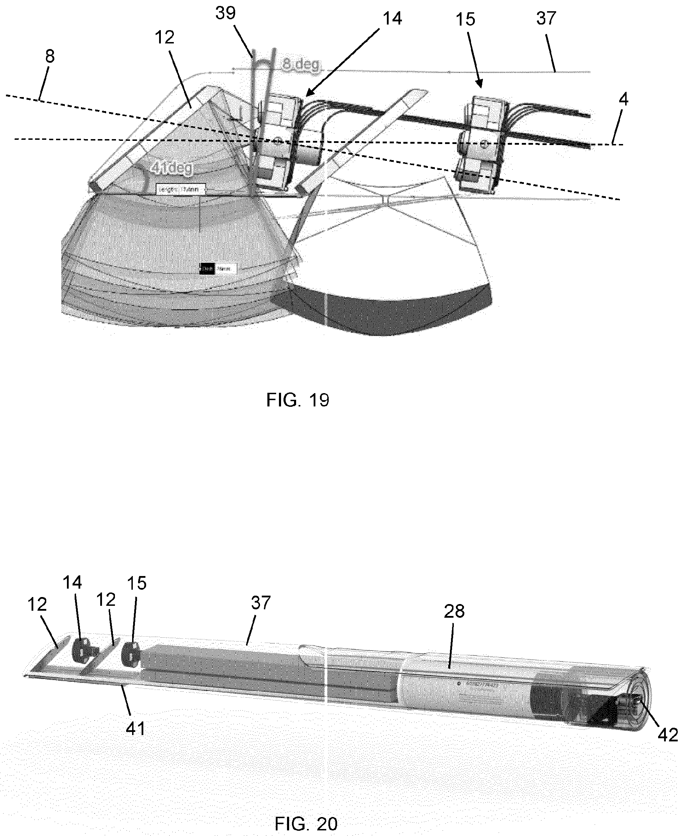

A reflecting element may be understood herein as an element configured to change the direction of light passing through or hitting the surface of said reflecting element. In particular, the reflecting element is preferably configured to change the direction of a center beam of the projected light from a projector unit from a direction substantially parallel to the longitudinal axis of the scanning device to a direction substantially orthogonal to said longitudinal axis. In some embodiments, a surface normal of each reflecting element defines an angle with respect to the projector optical axis of approximately 40-50 degrees, preferably approximately 45 degrees. This is exemplified in figures 3 and 4. In other embodiments, each scan unit defines an angle with respect to the longitudinal axis of the scanning device. As an example, the surface of the reflecting element may define an angle with respect to the longitudinal axis of between 40° to 45°, such as approximately 41°. This has the advantage of enabling a smaller tip height, e.g. compared to utilizing 45°, which is illustrated in figure 19.

As an example, the reflecting element may be selected from the group of: mirrors, prisms, and/or combinations thereof. In preferred embodiments, the reflecting element is configured to reflect light from the projector unit(s) of at least one scan unit and/or reflect light from the surface of object being scanned and onto the image sensor(s) of at least one scan unit. In some embodiments, at least one of the scan units comprises a mirror as the reflecting element. In other embodiments, at least one of the scan units comprises a prism as the reflecting element. Some embodiments feature a combination of mirror(s) and prism(s). In case of a prism as the reflecting element of at least one of the scan units, the prism is preferably configured to change the direction of a center beam of the projected light pattern from substantially parallel to the longitudinal axis of the scanning device to a direction having an angle of at least 45 degrees with respect to said longitudinal axis. Even more preferably, the prism is configured to change the direction of a center beam of the projected light pattern from substantially parallel to the longitudinal axis of the scanning device to a direction having an angle of approximately 90 degrees with respect to said longitudinal axis.

In some embodiments, the scanning device comprises a forward-looking scan unit, wherein the scanning device further comprises a reflecting element positioned on the projector optical axis of the projector unit of said scan unit. In other embodiments, the scanning device comprises at least two scan units, wherein each scan unit is configured as forward-looking, wherein each scan unit further comprises at least one reflecting element configured to reflect light from the projector unit(s) and/or reflect light from the object being scanned and onto the image sensor(s) of each camera of the scan unit. Thus, in some embodiments, each scan unit comprises a reflecting element. In some embodiments, the reflecting element(s) of the scan units are positioned on the projector optical axis. The projector optical axis may in some embodiments coincide with the longitudinal axis of the scanning device. The reflecting element(s) may be shaped in a variety of ways. As an example, each reflecting element may be substantially rectangular. In some embodiments, the reflecting element(s) comprise a plurality of corners, wherein at least some of the corners are rounded.

### Flexible cables

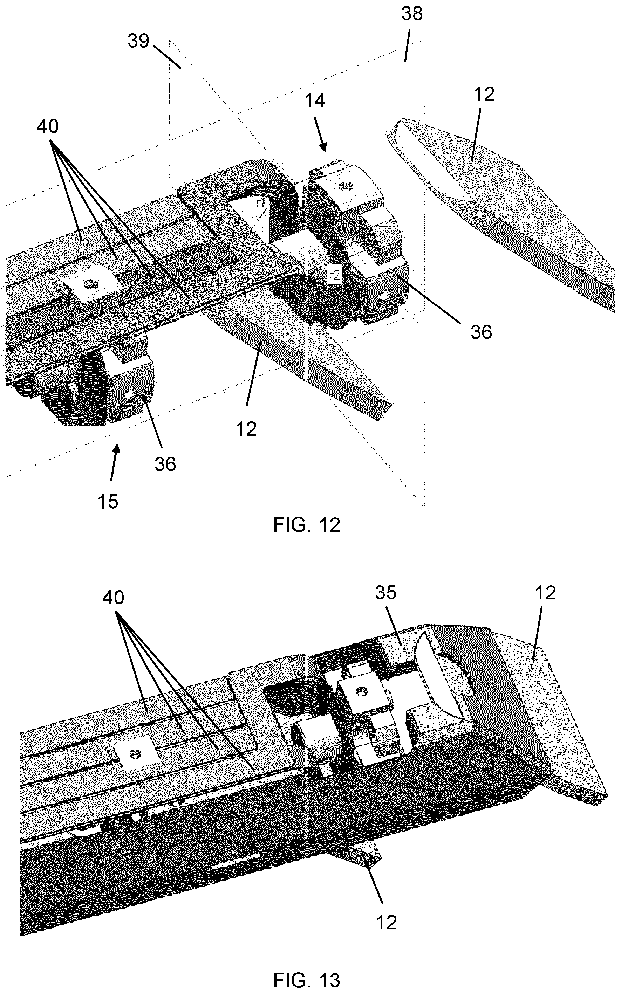

In some embodiments, the scanning device comprises one or more flexible cables, such as flexible printed circuits or flexible printed circuit boards (PCB). Each of the flexible cable may connect one of the cameras to a main printed circuit boards (PCB). Preferably, a flexible cable is connected to each camera, wherein each cable comprises a plurality of wires or circuits connected to the image sensor of a given camera. In some embodiments, each flexible cable is bent at a first radius of curvature, wherein the first radius of curvature lies in a first plane. Each cable may be further bent at a second radius of curvature, wherein the second radius of curvature lies in a second plane. Alternatively, each cable is a flexible PCB which is manufactured at a predetermined shape, such that each flexible PCB contains a first section which is straight, and a second section which includes one or more turns. Preferably, the second section includes three turns, which may be right-angle turns; however, the turns may have rounded corners. This is illustrated in figure 12. The first plane may be substantially parallel to the longitudinal axis of the scanning device, and the second plane may be perpendicular to the first plane. Thus, the manufactured flexible PCB may be bent only along the first radius of curvature, such that the entirety of the second section lies in the second plane. The first radius of curvature is selected from about 0.5 mm to about 5 mm. A first section of each cable may be substantially parallel to the longitudinal axis of the scanning device, and a second section of each cable may lie in the second plane. Furthermore, the cables may overlap each other along a section, wherein the cables are bent at the first radius of curvature. An advantage of providing the flexible cables in the arrangement described above and shown in figures 12-13 is that it ensures a low stress in each cable. In particular, this is the case if each flexible cable is bent along a first radius of curvature, wherein the radius is selected from about 0.5 mm to about 5 mm. Such a large radius of curvature ensures a minimum of stress in each cable.

### Processor

In accordance with some embodiments, the scanning device comprises one or more processors. The scanning device may comprise a first processor configured for determining image features in the acquired images. As an example, the first processor may be a field-programmable gate array (FPGA). The scanning device may further comprise computer memory for storing instructions, which when executed, causes the first processor to carry out the step of determining image features in the acquired images. The scanning device may further comprise a second processor configured for performing the steps of carrying out a computer-implemented method for generating a digital representation of a three-dimensional (3D) object. The digital representation may be generated continuously during a scanning session and it may be generated in real-time. As an example, the second processor may be configured for running a tracking algorithm for solving the correspondence problem, i.e. for determining corresponding image features in the obtained images.

The computer memory may further store instructions, which when executed, causes the second processor to carry out the method of generating a digital representation of a three-dimensional (3D) object. As an example, the second processor may be a central processing unit (CPU) such as an ARM processor or another suitable microprocessor. The second processor may comprise computer memory. The first and second processor may both be located on the scanning device, and they may be operatively connected such that the first processor provides input to the second processor. Alternatively, the first processor may be located on the scanning device, and the second processor may be located on the computer system described herein. As an example, the first processor may be configured to determine image features in the images, and subsequently provide data related to the determined image features to the second processor. The data may comprise image feature coordinates as well as other attributes such as a camera index or a predefined property, such as the phase, of the image feature(s).

The second processor may then be configured to generate the digital representation of the 3D object, e.g. in the form of a point cloud. The scanning device may be further configured to provide the digital representation to a computer system for rendering the representation. The computer system may further process the digital representation, e.g. by stitching the point clouds received from the scanning device and/or by fitting one or more surfaces to the stitched point clouds. This further processing by the computer system may also be referred to herein as reconstruction. The output of the reconstruction is a digital 3D model of the scanned object. The digital 3D model may be rendered and displayed on a display, e.g. connected to the computer system.

### Module for transmitting data

The scanning device preferably comprises a module for transmitting data, such as images or point clouds, to one or more external devices, such as a computer system. The module may be a wireless module configured to wirelessly transfer data from the scanning device to the computer system. The wireless module may be configured to perform various functions required for the scanning device to wirelessly communicate with a computer network. The wireless module may utilize one or more of the IEEE 802.11 Wi-Fi protocols/ integrated TCP/IP protocol stack that allows the scanning device to access the computer network. It may also use other network The wireless module may include a system-on-chip having different types of inbuilt network connectivity technologies. These may include commonly used wireless protocols such as Bluetooth, ZigBee, Wi-Fi, WiGig (also known as 60 GHz Wi-Fi), etc. The scanning device may further (or alternatively) be configured to transmit data using a wired connection, such as an ethernet cable or a USB cable. In some embodiments, the scanning device comprises a wireless module configured to wirelessly transfer data from the scanning device to the computer system.

### Computer system

A computer system may be understood as an electronic processing device for carrying out sequences of arithmetic or logical operations. In the present context, a computer system refers to one or more devices comprising at least one processor, such as a central processing unit (CPU), along with some type of computer memory. Examples of computer systems falling within this definition include desktop computers, laptop computers, computer clusters, servers, cloud computers, quantum computers, mobile devices such as smartphones and tablet computers, and/or combinations thereof.

The computer system may comprise hardware such as one or more central processing units (CPU), graphics processing units (GPU), neural processing units (NPU), and computer memory such as random-access memory (RAM) or read-only memory (ROM). The computer system may comprise a CPU, which is configured to read and execute instructions stored in the computer memory e.g. in the form of random-access memory. The computer memory is configured to store instructions for execution by the CPU and data used by those instructions. As an example, the memory may store instructions, which when executed by the CPU, cause the computer system to perform, wholly or partly, any of the computer-implemented methods disclosed herein. The computer system may further comprise a graphics processing unit (GPU). The GPU may be configured to perform a variety of tasks such as video decoding and encoding, rendering of the digital representation, and other image processing tasks.

The computer system may further comprise non-volatile storage in the form of a hard disc drive. The computer system preferably further comprises an I/O interface configured to connect peripheral devices used in connection with the computer system. More particularly, a display may be connected and configured to display output from the computer system. The display may for example display a 2D rendering of the generated digital 3D representation. The 2D rendering may be continuously generated and displayed in the display during a scanning session. Input devices may also be connected to the I/O interface. Examples of such input devices include a keyboard and a mouse, which allow user interaction with the computer system. A network interface may further be part of the computer system in order to allow it to be connected to an appropriate computer network so as to receive and transmit data (such as scan data and/or images) from and to other computing devices. The CPU, volatile memory, hard disc drive, I/O interface, and network interface, may be connected together by a bus.

The computer system is preferably configured for receiving data from the scanning device, either directly from the scanning device or via a computer network such as a wireless network. The data may comprise images, processed images, point clouds, sets of data points, or other types of data. The data may be transmitted/received using a wireless connection, a wired connection, and/or combinations thereof. The computer system may be configured for performing any of the computer-implemented methods disclosed herein, either fully or partly. In some embodiments, the computer system is configured for carrying out the computer-implemented method for generating a digital representation of a three-dimensional (3D) object as described herein. In some embodiments, the computer system is configured for receiving data, such as point clouds, from the scanning device and then subsequently perform the steps of reconstruction and rendering a digital representation of a three-dimensional (3D) object. Rendering may be understood as the process of generating one or more images from three-dimensional data. The computer system may comprise computer memory for storing a computer program, said computer program comprising computer-executable instructions, which when executed, causes the computer system to carry out the method of generating a digital representation of a three-dimensional (3D) object.

### Acquisition of images

In accordance with preferred embodiments, the scanning device is configured to acquire images of a three-dimensional (3D) object. The images are preferably acquired using a scanning device comprising one or more scan units, wherein each scan unit comprises a projector unit and one or more cameras. The scanning device may be an intraoral scanning device for acquiring images inside the oral cavity of a subject. The projector unit(s) of the scanning device are configured for projecting a predefined illumination pattern, such as a static pattern, onto a surface, e.g. onto the surface of the three-dimensional object. Once projected on the surface, some light will be reflected from the surface, which may then enter the camera(s) of the scanning device, whereby images of the 3D object can be acquired.

The images are preferably acquired using one or more cameras per projector unit, such as at least two cameras or at least four cameras for each projector unit. In preferred embodiments, each scan unit of the scanning device comprises a projector unit and four cameras. The images may be processed by a processor located on the scanning device, and then subsequently transmitted to the computer system. The images may also be transmitted, without any processing, to the computer system. In some embodiments, both raw images and processed images are transmitted by the scanning device to a computer system. In some embodiments, a processor located on the scanning device, receives the images as input and provides one or more point clouds as output by executing one or more steps of the disclosed computer-implemented method.

### Examples

According to one embodiment, the scanning device comprises two scan units, wherein the scan units are positioned in series along the longitudinal axis of the scanning device, preferably in the tip of the scanning device. This embodiment is illustrated in figure 3. It is noted that even though fig. 3 only displays two scan units, the scanning device may comprise other components as disclosed herein. In this embodiment, each scan unit is configured in a forward-looking configuration, i.e. wherein the projector unit of each scan unit projects light towards the distal end of the elongated probe of the scanning device. In other words, in the forward-looking configuration, the projector optical axis of each projector unit is substantially parallel to the longitudinal axis of the elongated probe. In this embodiment, each scan unit comprises one or more cameras, such as four cameras, each camera comprising an image sensor for acquiring images, wherein the at least one camera defines a camera optical axis. In this embodiment, each scan unit further comprises a reflecting element, such as a mirror, configured to reflect light from the projector unit of a given scan unit and/or reflect light from the object being scanned and onto the image sensor(s) of each camera of the scan unit. According to this embodiment, the scanning device comprises a first scan unit and a second scan unit, wherein the first scan unit is positioned further towards the distal end of the probe than the second scan unit. The two scan units may be configured to project the light pattern at two different focus distances, such that the first scan unit is configured to project the light pattern at a predefined first focus distance, and the second scan unit is configured to project the light pattern at a predefined second focus distance. In some embodiments, the second focus distance is larger than the first focus distance.

Preferably, the two scan units are positioned as close as possible without any light rays projected by the first scan unit are cut off by the reflecting element of the second scan unit. As an example of how to describe the compactness of the scan units, a minimum bounding box of the first scan unit may overlap a minimum bounding box of the second scan unit. A bounding box may be understood as a virtual bounding box, in which the entirety of the scan unit can fit. A minimum bounding box may be understood as a virtual bounding box with the smallest area and/or volume within which the entirety of the scan unit can fit. Thus, a bounding box may be understood as a virtual tool for describing the outer perimeter of a scan unit, when said scan unit is enclosed in a box, the box being a rectangular cuboid. Thus, in some embodiments, a bounding box of a first scan unit overlaps a bounding box of a second scan unit.

According to another embodiment, the scanning device comprises two scan units, a first and a second scan unit, the first scan unit comprising a first projector unit configured to project the light pattern at a predefined first focus distance, and the second scan unit comprising a second projector unit configured to project the light pattern at a predefined second focus distance, wherein the scan units are positioned in series along the longitudinal axis of the scanning device, preferably in the tip of the scanning device, and wherein the first scan unit is configured in a forward-looking configuration and the second scan unit is configured in a downward-looking configuration. Accordingly, the projector optical axis of the first projector unit is substantially parallel with the longitudinal axis of the scanning device, and the projector optical axis of the second projector unit is substantially orthogonal to the longitudinal axis of the scanning device. This embodiment is illustrated in figure 7. In this embodiment, each scan unit comprises one or more cameras, such as four cameras, each camera comprising an image sensor for acquiring images, wherein the at least one camera defines a camera optical axis. In this embodiment, the first scan unit further comprises a reflecting element, such as a mirror or prism, the reflecting element being configured to reflect light from the first projector unit and/or reflect light from the surface of the object being scanned and onto the image sensor(s) of the first scan unit.

The two annulus sectors shown below the scanning device in fig. 7 illustrates scan volumes, i.e. a volume which is observable by the scanning device. Accordingly, a first scan volume is associated with the first scan unit and a second scan volume is associated with the second scan unit. The first and second scan volumes may overlap in time and/or space. An advantage hereof is that the accuracy of the generation of the 3D representation may be improved. Furthermore, the two scan volumes may be projected at two different distances below the tip of the scanning device as illustrated in fig. 7. This can be achieved in at least two different ways. If the same optical components are used, i.e. if the first and second projector units are similar in configuration, the first and second focus distance will be similar when measured along each projector optical axis, respectively. However, since the first and second scan units, including projector units, are oriented differently, the two scan unit will project the light pattern at two different distances below the tip of the scanning device. In particular, this is the case if more of the optical path lie inside a housing of the scanning device, such as illustrated for the first (most distal) scan unit, whereas for the second scan unit, the optical path inside the housing is shorter, since the light is projected directly down. Alternatively, if different optical components are used, the two different distances below the scanning device may be achieved by having different focus distances for the first and second projector units, i.e. such that the second focus distance is larger than the first focus distance.

An advantage of the embodiment shown in fig. 7 is that the two scan volumes are projected at two different distances, such that the first scan unit projects the light at a first distance below the tip of the scanning device, and the second scan unit projects the light at a second distance below the tip of the scanning device, wherein the second distance is larger than the first. This is particularly useful since some users hold the scanning device at an angle during scanning, wherein the most distal part of the scanning device is closer to the dental object being scanned compared to less distal parts of the scanning device.

### Detailed description of the drawings

Fig. 1 shows a side-view (cross-sectional view) of a scan unit **5** comprising a projector unit **6** and four cameras **9,** wherein the scan unit is configured to project light directly down on the dental object **2.** This configuration of the scan unit is also referred to herein as 'downward-looking'. The longitudinal axis **4** of the scanning device **1** is horizontal in this figure.

Fig. 2 shows a perspective view of a scan unit **5** comprising a projector unit **6** and four cameras **9,** wherein the projector unit is configured to project a light pattern **13** (here a checkerboard pattern) onto a surface of the dental object. The light pattern may be a static pattern in time.

Fig. 3 shows a side-view (cross-sectional view) of two scan units **5** positioned in series along the longitudinal axis **4** of the scanning device **1.** In this embodiment, both of the two scan units comprise a projector unit **6,** four cameras **9,** and a reflecting element **12** (such as a mirror) configured to direct (a center beam of) the light from a substantially horizontal direction to a substantially orthogonal direction with respect to the longitudinal axis **4** of the scanning device. The many thin lines illustrate projected light rays / reflected light rays from the surface.

Fig. 4 shows a side-view (cross-sectional view) of a scan unit **5** comprising a projector unit **6,** four cameras **9,** and a reflecting element **12,** such as a mirror, wherein some dimensions are included on the drawing. In this figure, 'a' denotes the baseline, which may be understood as the center-to-center distance between a given camera **9** and the projector unit **6,** 'b' denotes the distance from the projector optical axis **8** to the outer edge of the scan unit **5** as illustrated in the figure, 'c' denotes the distance between the projector unit **6** and the reflecting element **12,** said distance measured along the projector optical axis **8,** 'd' denotes the length of the scan unit **5,** and 'e' denotes the camera-projector angle, which is the angle between a given camera optical axis **11** and the projector optical axis **8.** Generally, the intersection of two straight lines, such as the two aforementioned axes, may give rise to two angles. The camera-projector angle may be understood as the smallest of said angles.

Fig. 5A shows a side-view of a scanning device **1** comprising two scan units **5,** such that the scanning device has an extended field of view along the longitudinal axis **4** ('long FOV' configuration) compared to a scanning device comprising one scan unit. An advantage hereof is that the field of view is larger (compared to one scan unit), which enables more data/images to be captured per unit of time, which allows a 3D representation of the scanned object to be generated faster. Additionally, a large field of view allows for smaller registration errors and consequently a better global accuracy of the 3D representation. The cones **34** illustrate the projected light from the scan units.

Fig. 5B shows the scanning device of fig. 5A seen from the distal end of the scanning device.

Fig. 5C shows a side-view of a scanning device **1** comprising two scan units **5,** such that the scanning device has an extended field of view ('double swipe' configuration) compared to a scanning device comprising one scan unit. In this embodiment, the two scan units are angled with respect to each other such that they illuminate different parts of the dental object **2** from different angles, cf. fig. 5D. An advantage hereof is that more surface area of the dental object is illuminated per unit of time, thus enabling the entirety of the dental object to be scanned faster during a scanning session with less movements of the scanning device. Another advantage is that the parts/areas of the dental object are illuminated from different angles, thus increasing the accuracy of the generation of the 3D representation based on the images obtained of the dental object. The cone **34** illustrates the projected light from the scan units.

Fig. 5D shows the scanning device of fig. 5C seen from the distal end of the scanning device. The cones **34** illustrate the projected light from the scan units, said scan units projecting the light at a predefined angular field of view. It is noted that in this configuration, the scan units **5** are angled, such that the projector optical axes **8** of the two scan units are angled with respect to each other, such the two scan units illuminate the dental object **2** from different angles.

Fig. 5E shows a side-view of a scanning device **1** comprising two scan units **5,** such that the scanning device has an extended field of view ('single swipe' configuration) compared to a scanning device comprising one scan unit. In this embodiment, the two scan units **5** are angled with respect to each other such that they illuminate different parts of the dental object **2** from different angles, cf. fig. 5F. In this embodiment, the angle is measured in a plane perpendicular to the longitudinal axis of the scanning device. The cone **34** illustrates the projected light from the scan units.

Fig. 5F shows the scanning device 1 of fig. 5E seen from the distal end of the scanning device. This embodiment is largely similar to the embodiment shown in fig. 5C-5D, except in this case the angle between the projector optical axes **8** of the two scan units **5** is larger compared to the embodiment of fig. 5D. In this embodiment, the angle is measured in a plane perpendicular to the longitudinal axis of the scanning device. The angle may be larger than 45°, such as larger than larger than 75°, such as larger than 85°. As an example, the angle may be approximately 90° as illustrated in fig. 5F. An advantage of a large angle between the projector optical axes is that the scanning device **1** can illuminate the dental object **2** from different angles, preferably such that the entirety of a tooth is within the field of view of the scanning device. Thereby, less movements of the scanning device are necessary during the scanning session, since a greater surface area of the dental object is covered/captured per unit of time.

Fig. 6 shows a dental scanning system comprising an intraoral scanning device **1** and a computer system **26,** here a laptop. In preferred embodiments, the scanning device **1** is configured for wirelessly transmitting scan data, such as images or 3D data, to the computer **26.** Scan data may comprise image(s), processed image(s), 2D data, 3D data, processed 3D data, 3D representation(s), fluorescence data, infrared (IR) data, near-infrared (NIR) data, and/or combinations thereof. The scan data may be transmitted by a wireless module **31** configured to wirelessly transfer data from the scanning device to the computer system. Alternatively, the scanning device may be configured to transfer scan data using a wired connection **32.** The computer system **26** may comprise a display for displaying a 3D representation **27** or a 3D model based on the 3D representation. The scanning device may be configured to transmit the scan data in real-time to an external processing unit, such as the computer system disclosed herein.

Fig. 7 shows a side-view of a scanning device **1,** in particular the tip **25** of the scanning device, wherein the scanning device comprises two scan units **5** in two different configurations. In this embodiment, a first scan unit **14** is configured in a forward-looking configuration, i.e. such that the projector unit projects the light in a forward direction along an optical path substantially parallel with the longitudinal axis of the scanning device. The first scan unit further comprises a mirror **12** configured to direct (a center beam of) the light from a substantially horizontal direction to a substantially orthogonal direction with respect to the longitudinal direction of the scanning device. A second scan unit **15** is configured in a downward-looking configuration, i.e. such that the projector unit of this scan unit projects the light in a downward direction, i.e. substantially orthogonal to the longitudinal axis of the scanning device. An advantage of this embodiment is that the first scan unit may project the light at a predefined first distance below the tip of the scanning device, and the second scan unit may project the light at a predefined second distance below the tip of the scanning device, wherein the second distance is larger than the first. This is particularly useful since some users hold the scanning device at an angle during scanning. Accordingly, the first scan unit **14** may be configured to project a first scan volume **16** at a first distance below the tip **25** of the scanning device **1,** and the second scan unit **15** may be configured to project a second scan volume **17** at a second distance below the tip **25** of the scanning device **1.** The first **16** and second scan volumes **17** may overlap in time and/or space.

Fig. 8A-8D show four different ways in which the projector unit **6** and cameras **9** of a scan unit **5** can be positioned/oriented. In these embodiments, the image sensors **10,** optical masks **18,** and reflecting elements **12,** each comprises four edges, said edges forming a rectangle. Some of the corners of the rectangle(s) may be rounded. In some embodiments, the reflecting element **12** has a rectangular shape, wherein some of the corners of the rectangle are rounded. Furthermore, in this embodiment, for each scan unit **5,** one or more edges of the image sensor **10,** optical mask **18,** and reflecting element **12** are substantially aligned, such that corresponding edges are substantially parallel. As seen from fig. 8A, the edges of the image sensors **10** are parallel with the edges of the reflecting element **12,** when viewed in this cross-section.

In fig. 8A the cameras **9** are arranged on two axes to form a cross in a plane, wherein the projector unit **6** is located in the center of the cross when viewed along the projector optical axis **8.** Thus, a first camera **9** is located above the projector unit **6** in the plane, a second camera **9** is located below the projector unit **6** in the plane, a third camera **9** is located to the left of the projector unit **6** in the plane, and a fourth camera **9** is located to the right of the projector unit **6** in the plane. The two axes of the cross are substantially parallel with at least two edges of the reflecting element **12.** In fig. 8B, the cross is rotated 45 degrees with respect to the edges/sides of the reflecting element **12.** In other words, the diagonals of the optical mask **18 /** image sensors **10** are aligned with a vertical axis of the reflecting element **12.** Fig. 8C shows an example, wherein the cameras **9** and projector unit **6** are arranged in a cross as in fig. 8B, but wherein the edges of the projector unit **6** are substantially aligned with the edges of the reflecting element **12.** Fig. 8D show an example, which is similar to the example of fig. 8C, but wherein the cameras **9** are rotated such that the edges of the image sensors **10** are substantially aligned with the edges of the reflecting element **12.**

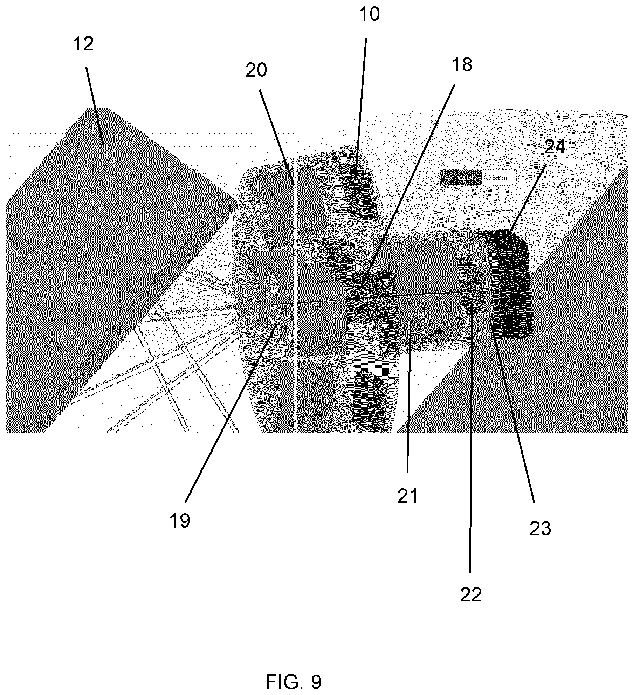

Fig. 9 shows a perspective view of a scan unit **5** comprising a projector unit **6** and four cameras **9,** wherein the scan unit further comprises a reflecting element **12,** here shown as a mirror. The projector unit **6** may comprise a light source **22,** an optical mask **18** for defining a pattern, one or more optical lenses **(19, 21).** Each camera comprises an image sensor **10** and may further comprise one or more camera lenses **20.**

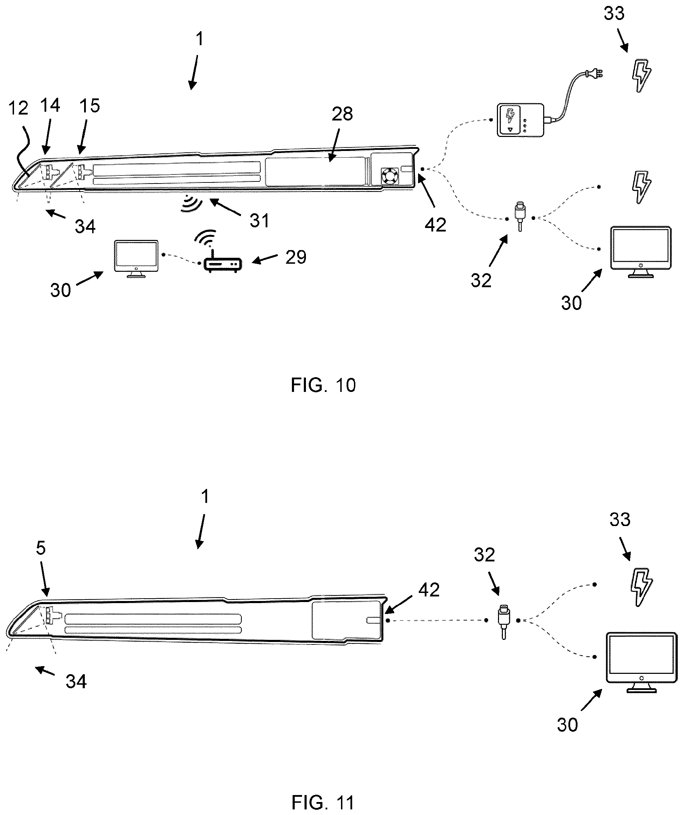

Fig. 10 shows an embodiment of a scanning device **1** comprising two scan units **5,** wherein each scan unit **5** comprises a reflecting element **12,** here exemplified as a mirror. In this embodiment, the reflecting element **12** is configured to alter the direction of a (center beam of) the projected light **34** from a direction substantially parallel to the longitudinal axis **4** of the scanning device **1** to a direction substantially orthogonal to said longitudinal axis **4.** In this embodiment, the scanning device further comprises a wireless module **31** configured to wirelessly transfer scan data, such as images, 3D data or other data, to a computer system **26,** e.g. via a Wi-Fi router **29** or a Wi-Fi access point. The scan data may be transferred continuously and/or in real-time. The computer system **26** may comprise a display **30** for displaying a 3D representation of the dental object. Furthermore, the scanning device may comprise a battery **28** for powering the scanning device.

Fig. 11 shows an embodiment of a scanning device **1** comprising one scan unit **5,** said scan unit comprising a reflecting element **12,** here exemplified as a mirror. In this embodiment, the scanning device is configured to transfer scan data using a wired connection **32,** such as a USB cable or ethernet cable. Furthermore, the scanning device may be powered by a wired connection **32** to a power supply **33.** The scanning device may transfer data to a computer system **26** comprising a display **30** for displaying a 3D representation of the dental object.

Fig. 12 shows an embodiment of a scanning device comprising a first scan unit **14** and a second scan unit **15,** wherein each scan unit further comprises a reflecting element **12,** here a mirror. Thus, a first reflecting element is associated with the first scan unit, and a second reflecting element is associated with the second scan unit. In this figure, some elements of the scanning device have been omitted to highlight other elements. In particular, the outer shell and the frame has been removed, such that the scan units, reflecting elements, and flexible cables **40** are visible. In this embodiment, four flexible printed circuit boards (PCB) **40** are shown, wherein each flexible PCB are connected to an image sensor of a camera. A fifth flexible PCB is connected to the projector unit. The figure further defines two planes, a first plane **38** and a second plane **39.** In this embodiment, each flexible PCB is bent at a first radius of curvature, wherein the first radius of curvature lies in the first plane **38.** Furthermore, each flexible PCB is manufactured to have a certain predetermined shape, wherein a second section includes one or more turns. Each flexible PCB is then bent such that the second section lies in a second plane **39.** In this example, the first plane is substantially parallel to the longitudinal axis of the scanning device, and the second plane is perpendicular to the first plane. Notice that the second scan unit **15** intersects with some of the flexible PCBs; this is merely an artifact in the rendering and therefore does not accurately represent the physical device.

Fig. 13 shows the same embodiment as shown in fig. 12. In this visualization, a frame **35** of the scanning device is shown. The frame is configured to accommodate the scan units **(14, 15).** The frame is further configured such that the reflecting elements **12** can be inserted and mounted in the frame. Notice that the reflecting elements **12** may protrude from the frame, when mounted. The frame may comprise one or more openings for inserting the scan units.

Fig. 14 shows the same embodiment as shown in fig. 13; here shown as an exploded view. The frame **35** may be made in one piece, and is preferably manufactured to be rigid. Examples of suitable materials for the frame includes: aluminium, steel, stainless steel, a magnesium alloy, a polymer, or an iron-carbon alloy. The frame **35** may comprise two or more openings for receiving the scan units **(14, 15).** In this embodiment, the frame comprises a first opening for receiving the first scan unit **14,** and a second opening for receiving the second scan unit **15.** In this embodiment, the frame is configured such that the scan units may be inserted in the frame from opposite directions, e.g. the first scan unit from above the frame, and the second scan unit from below the frame. Accordingly, the scanning device may be assembled by inserting the first scan unit **14** into the frame **35** through a first opening located in a first surface of the frame; and inserting the second scan unit **15** into the frame **35** through a second opening located in a second surface of the frame. The reflecting elements **12** may be inserted in the frame from below. The frame **35** may be configured such that the reflecting elements **12** define an angle with respect to the longitudinal axis of the scanning device when mounted.

Fig. 15 shows the same embodiment as shown in figures 12-14, except that in this figure both the frame **35,** and an outer shell **37,** as well as an optical window **41** of the scanning device is visualized. This figure shows a cross-section of the distal part of the scanning device, when the scan units **(14, 15)** and reflecting elements **12** have been mounted in the frame **35,** and when the said elements and frame are housed inside the outer shell **37** of the scanning device. The optical window **41** may be flush with the surface of the outer shell **37.**

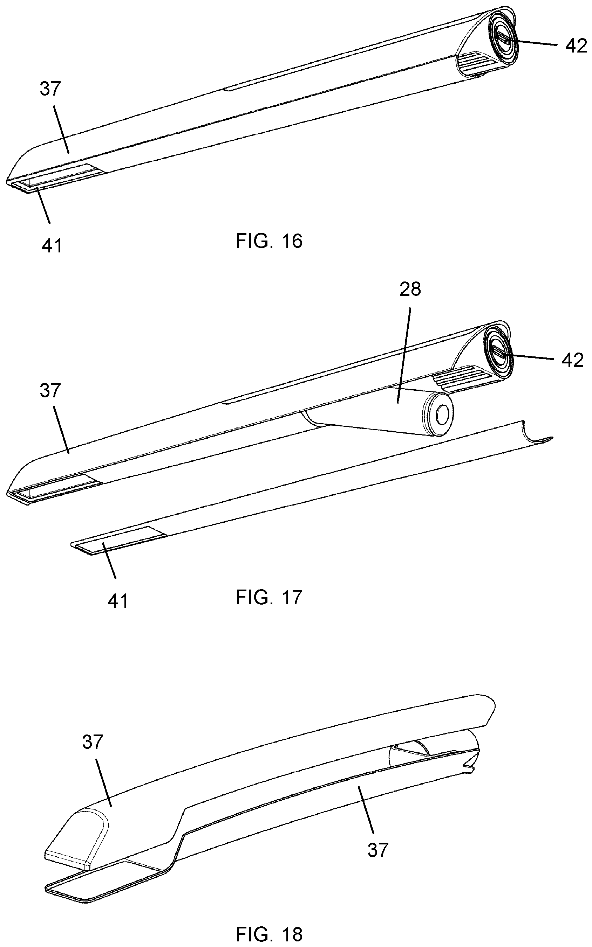

Fig. 16 shows a perspective view of an intraoral scanning device according to the present disclosure. The outer shell **37** may comprise a replaceable part, which may be removably mounted to the remaining part of the outer shell. The replaceable part may further comprise an optical window **41,** e.g. placed near the distal end of the scanning device when mounted. The scanning device may further comprise a port **42** configured for receiving a cable, such as a data cable or a power cable for charging an internal power supply, e.g. a battery, inside the scanning device. In some embodiments, the port is configured for receiving a data cable, or a combined data- and power cable.

Fig. 17 shows a perspective view of the same embodiment shown in fig. 16; however, in this view, the replaceable part is shown detached from the scanning device. In this embodiment, the replaceable part comprises an optical window **41.** The window is located in the replaceable part, such that when said part is mounted to the remaining part of the outer shell, the optical window **41** aligns with the opening in said shell, such that projected light from the scanning device may exit the opening and be transmitted through the optical window. An advantage of placing the optical window **41** in a replaceable part of the outer shell **37** is that the window may easily be replaced e.g. if the window is damaged or scratched. The replaceable part may further be configured to cover a compartment for housing a battery **28.** Thus, a further advantage of the replaceable part is that a user may easily access said compartment for replacing the battery **28.** In some embodiments, the battery is a rechargeable battery, which may be recharged using a cable inserted in port **42.**

Fig. 18 shows a perspective view of an intraoral scanning device according to the present disclosure. In this embodiment, the scanning device comprises an outer shell 37, which comprises two replaceable parts, which may be assembled and/or disassembled. In this embodiment, a first replaceable part of the outer shell may be configured to cover a top part of the scanning device, and a second replaceable part of the outer shell may be configured to cover a bottom part of the scanning device. Similar to the embodiment shown in fig. 17, the replaceable part, e.g. the second replaceable part, may comprise an optical window. Furthermore, the user may access a compartment for housing a battery as explained in relation to fig. 17.

Fig. 19 shows a cross-sectional view of an embodiment of an intraoral scanning device according to the present disclosure. In this embodiment, the scanning device comprises two scan units **(14, 15),** each arranged in combination with a mirror **12.** In this embodiment, each scan unit is angled with respect to the longitudinal axis 4 of the scanning device and/or with respect to a vertical plane **39,** which is orthogonal to said longitudinal axis. As an example, each mirror **12** may define an angle of between 35° to 45°, such as between 40° to 45°, with respect to the longitudinal axis **4** of the scanning device. In this example, the angle is measured between a planar surface of the mirror and the longitudinal axis. Similarly, the projector- and cameras of each scan unit may be angled with a similar angle such that the relative orientation between the projector unit and mirror is maintained. Thus, in some embodiments the projector optical axis **8** defines an angle with the longitudinal axis **4** of between 0° to 12°, such as between 5° to 10°, such as approximately 8°. An advantage of such an arrangement, wherein each scan unit is angled with respect to the longitudinal axis, is that the tip height of the scanning device can be made smaller for the same optical system. A small tip height is desired, since it is more comfortable for the patient, e.g. when scanning inside the patient's mouth, but it also makes it easier to scan hard-to-reach areas, such as areas in the back of the patient's mouth.

Fig. 20 shows a perspective view of an intraoral scanning device according to the present disclosure. For illustrative purposes, the outer shell **37** is made transparent in the figure in order to visualize the components inside the scanning device. In this embodiment, the scanning device comprises a first scan unit **14** and a second scan unit **15,** wherein both scan units are arranged in combination with a reflecting element **12,** here exemplified as a mirror. The scanning device defines or comprises an elongated probe having a longitudinal axis. The optical axis of the projector units of each scan unit **(14, 15)** may be substantially parallel to the longitudinal axis at least until contact with the reflecting element **12,** which is preferably arranged to redirect light rays projected by the projector units through the optical window **41** and onto a surface of the object being scanned. In this embodiment, the scanning device further comprises an internal power supply **28,** such as a battery, and a port **42** for charging said power supply and/or for providing wired data transfer via a cable (not shown).

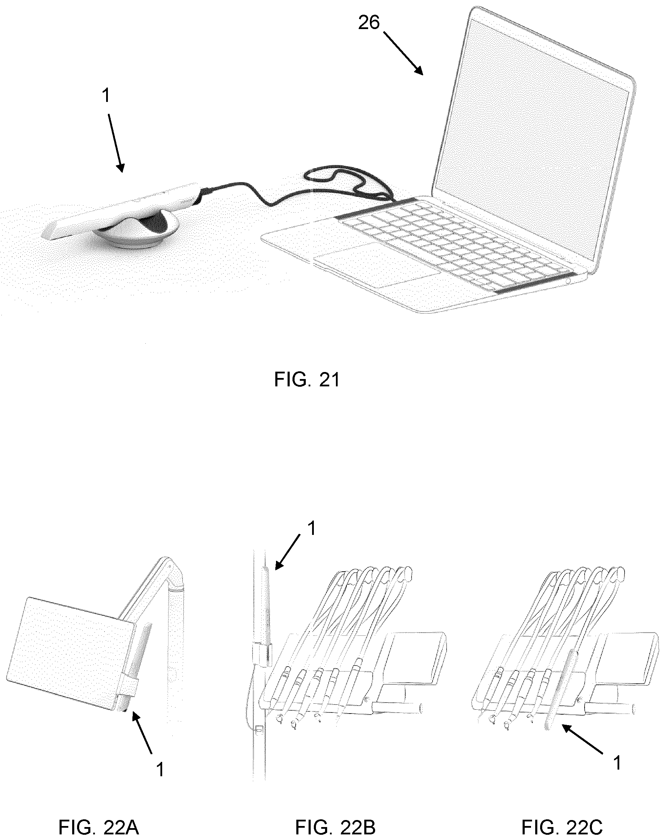

Fig. 21 shows an embodiment of a dental scanning system comprising an intraoral scanning device **1** and a computer system **26,** here exemplified as a laptop. In this embodiment, the scanning device is configured to connect to the computer system via a wired connection. The dental scanning system may be configured to transfer data and/or power via the wired connection. In other embodiments, the scanning device **1** is configured to wirelessly transfer data, such as images and/or scan data, to the computer system **26.** The dental scanning system may further comprise a display for displaying a 3D representation of the scanned object. In this example, the laptop comprises a display.

Fig. 22 shows three different embodiments of how to provide a chairside solution of a dental scanning system according to the present disclosure. Fig. 22A shows a moveable cart with a display mounted on a mechanical arm. The cart includes a holder next to the display for holding the intraoral scanning device **1.** Fig. 22B shows the scanning device in the holder placed near a power outlet for charging the scanning device. Fig. 22C shows the scanning device placed next to other typical dental instruments for complete chair integration.

Although some embodiments have been described and shown in detail, the disclosure is not restricted to such details, but may also be embodied in other ways within the scope of the subject matter defined in the following claims. In particular, it is to be understood that other embodiments may be utilized, and structural and functional modifications may be made without departing from the scope of the invention which is defined by the appended claims.

A claim may refer to any of the preceding claims, and "any" is understood to mean "any one or more" of the preceding claims.

It should be emphasized that the term "comprises/ comprising/ including" when used in this specification is taken to specify the presence of stated features, integers, operations, steps or components but does not preclude the presence or addition of one or more other features, integers, steps, components or groups thereof.

### Reference numerals

1. Intraoral scanning device
2. Dental object
3. Elongated probe
4. Longitudinal axis
5. Scan unit
6. Projector unit
7. Light pattern
8. Projector optical axis
9. Camera
10. Image sensor
11. Camera optical axis
12. Reflecting element
13. Light pattern
14. First scan unit
15. Second scan unit
16. First scan volume
17. Second scan volume
18. Mask
19. Focus lens
20. Camera lens
21. Collimation lens
22. Light source
23. Printed circuit board (PCB)
24. Heat sink
25. Tip of scanning device
26. Computer system
27. 3D representation
28. Battery
29. Wi-Fi router
30. Display
31. Wireless module
32. Wired connection
33. Power supply
34. Projected light / scan volume
35. Frame
36. Housing
37. Outer shell
38. First plane
39. Second plane
40. Flexible cable / flexible PCB
41. Optical window
42. Port for charging and/or data transmission

## Claims

1. An intraoral scanning device (1) for scanning a dental object (2), comprising:
- an elongated probe (3) defining a longitudinal axis (4) of the scanning device (1);
- a first scan unit (14) comprising:
- a first projector unit configured to project a light pattern (7) onto a surface of the dental object (2), wherein the first projector unit defines a first projector optical axis;
- at least one camera (9) comprising an image sensor (10) for acquiring images, wherein the at least one camera (9) defines a camera optical axis (11); and
- a reflecting element (12) configured to reflect light from the first projector unit and/or reflect light from the surface of the dental object (2) and onto the image sensor(s) (10) of each camera (9);
- a second scan unit (16) comprising:
- a second projector unit configured to project a light pattern (7) onto a surface of the dental object (2), wherein the second projector unit defines a second projector optical axis; and
- at least one camera comprising an image sensor for acquiring images, wherein the at least one camera defines a camera optical axis;
wherein each scan unit (14; 16) defines a field of view (FOV),
**characterized in that**
the projector optical axis and the camera optical axis of each scan unit define a camera-projector angle of between 5° to 10°.

2. The scanning device according to claim 1, wherein the projector unit is configured to project unpolarized white light.

3. The scanning device according to any of the preceding claims, wherein the light pattern is static in time.

4. The scanning device according to any of the preceding claims, wherein the scanning device comprises an optical window made of a polymer or glass, wherein the optical window is located in a distal end of the elongated probe.

5. The scanning device according to any of the preceding claims, wherein the first and second scan units are arranged to project the light pattern through the optical window.

6. The scanning device according to any of the preceding claims, wherein the second scan unit further comprises a reflecting element configured to reflect light from the second projector unit and/or reflect light from the surface of the dental object and onto the image sensor(s) of each camera of the second scan unit.

7. The scanning device according to any of the preceding claims, wherein the scan units are positioned along the longitudinal axis of the scanning device.

8. The scanning device according to any of the preceding claims, wherein the first and second projector optical axes are substantially parallel.

9. The scanning device according to any of the preceding claims, wherein a minimum bounding box of a first scan unit overlaps a minimum bounding box of a second scan unit.

10. The scanning device according to any of the preceding claims, wherein the first and second scan unit are configured to project the light pattern on the same side of the scanning device.

11. The scanning device according to any of the preceding claims, wherein each scan unit comprises two or more cameras, wherein said cameras are configured to acquire a set of images.

12. The scanning device according to claim 11, wherein the number of images in the set of images corresponds to the number of cameras in a given scan unit.

13. The scanning device according to any of the claims 11-12, wherein the cameras are synchronized such that images within the set of images are acquired simultaneously.

14. The scanning device according to any of the preceding claims, wherein each scan unit comprises four cameras.

15. The scanning device according to any of the preceding claims, wherein each scan unit comprises one projector unit and a plurality of cameras, wherein the cameras are arranged on two axes to form a cross in the plane, wherein the projector unit is located in the center of the cross when viewed along the projector optical axis, wherein the two axes are substantially parallel with at least two edges of the reflecting element.

16. The scanning device according to any of the preceding claims, wherein each scan unit defines an angular field of view (AFOV) of between 60° to 75°.

17. The scanning device according to any of the preceding claims, wherein for a given scan unit, the center-to-center distance between the projector unit and a given camera of that scan unit is between 2 to 5 mm, preferably between 3 to 4 mm.

18. The scanning device according to any of the preceding claims, wherein the field of view of each scan unit is at least 400 mm² at a working distance of between 15 mm and 50 mm.

19. The scanning device according to any of the preceding claims, wherein the scanning device employs a triangulation-based scanning principle.

20. The scanning device according to any of the preceding claims, wherein the scanning device comprises one or more processors configured for generating a three-dimensional (3D) representation of the dental object.

## Patentansprüche

1. Intraorale Scanvorrichtung (1) zum Scannen eines dentalen Objekts (2), umfassend:
- eine längliche Sonde (3), die eine Längsachse (4) der Scanvorrichtung (1) definiert;
- eine erste Scaneinheit (14), umfassend:
- eine erste Projektoreinheit, die zur Projektion eines Lichtmusters (7) auf eine Oberfläche des dentalen Objekts (2) eingerichtet ist, wobei die erste Projektoreinheit eine erste optische Projektorachse definiert;
- mindestens eine Kamera (9), umfassend einen Bildsensor (10) zum Aufnehmen von Bildern, wobei die mindestens eine Kamera (9) eine optische Kameraachse (11) definiert; und
- ein reflektierendes Element (12), das zum Reflektieren von Licht von der ersten Projektoreinheit und/oder zum Reflektieren von Licht von der Oberfläche des dentalen Objekts (2) und auf den einen oder die mehreren Bildsensoren (10) jeder Kamera (9) eingerichtet ist;
- eine zweite Scaneinheit (16), umfassend:
- eine zweite Projektoreinheit, die zur Projektion eines Lichtmusters (7) auf eine Oberfläche des dentalen Objekts (2) eingerichtet ist, wobei die zweite Projektoreinheit eine zweite optische Projektorachse definiert; und
- mindestens eine Kamera, umfassend einen Bildsensor zum Aufnehmen von Bildern, wobei die mindestens eine Kamera eine optische Kameraachse definiert;
wobei jede Scaneinheit (14; 16) ein Sichtfeld (FOV) definiert, **dadurch gekennzeichnet, dass** die optische Projektorachse und die optische Kameraachse jeder Scaneinheit einen Kamera-Projektor-Winkel von zwischen 5° und 10° definieren.

2. Scanvorrichtung nach Anspruch 1, wobei die Projektoreinheit eingerichtet ist, unpolarisiertes weißes Licht zu projizieren.

3. Scanvorrichtung nach einem der vorhergehenden Ansprüche, wobei das Lichtmuster zeitlich statisch ist.

4. Scanvorrichtung nach einem der vorhergehenden Ansprüche, wobei die Scanvorrichtung ein optisches Fenster aus einem Polymer oder Glas umfasst, wobei sich das optische Fenster an einem distalen Ende der länglichen Sonde befindet.

5. Scanvorrichtung nach einem der vorhergehenden Ansprüche, wobei die erste und die zweite Scaneinheit so ausgelegt sind, dass sie das Lichtmuster durch das optische Fenster projizieren.

6. Scanvorrichtung nach einem der vorhergehenden Ansprüche, wobei die zweite Scaneinheit ferner ein reflektierendes Element umfasst, das zum Reflektieren von Licht von der zweiten Projektoreinheit und/oder zum Reflektieren von Licht von der Oberfläche des dentalen Objekts und auf den einen oder die mehreren Bildsensoren jeder Kamera der zweiten Scaneinheit eingerichtet ist.

7. Scanvorrichtung nach einem der vorhergehenden Ansprüche, wobei die Scaneinheiten entlang der Längsachse der Scanvorrichtung positioniert sind.

8. Scanvorrichtung nach einem der vorhergehenden Ansprüche, wobei die erste und die zweite optische Projektorachse im Wesentlichen parallel sind.

9. Scanvorrichtung nach einem der vorhergehenden Ansprüche, wobei ein Mindestbegrenzungsrahmen einer ersten Scaneinheit einen Mindestbegrenzungsrahmen einer zweiten Scaneinheit überlappt.

10. Scanvorrichtung nach einem der vorhergehenden Ansprüche, wobei die erste und die zweite Scaneinheit eingerichtet sind, das Lichtmuster auf die gleiche Seite der Scanvorrichtung zu projizieren.

11. Scanvorrichtung nach einem der vorhergehenden Ansprüche, wobei jede Scaneinheit zwei oder mehr Kameras umfasst, wobei die Kameras eingerichtet sind, einen Satz von Bildern aufzunehmen.

12. Scanvorrichtung nach Anspruch 11, wobei die Anzahl der Bilder in dem Satz von Bildern der Anzahl der Kameras in einer gegebenen Scaneinheit entspricht.

13. Scanvorrichtung nach einem der Ansprüche 11 bis 12, wobei die Kameras so synchronisiert sind, dass Bilder innerhalb des Satzes von Bildern gleichzeitig aufgenommen werden.

14. Scanvorrichtung nach einem der vorhergehenden Ansprüche, wobei jede Scaneinheit vier Kameras umfasst.

15. Scanvorrichtung nach einem der vorhergehenden Ansprüche, wobei jede Scaneinheit eine Projektoreinheit und eine Mehrzahl von Kameras umfasst, wobei die Kameras auf zwei Achsen angeordnet sind, um in der Ebene ein Kreuz zu bilden, wobei die Projektoreinheit im Zentrum des Kreuzes angeordnet ist, wenn entlang der optischen Projektorachse betrachtet wird, wobei die zwei Achsen im Wesentlichen parallel zu mindestens zwei Kanten des reflektierenden Elements sind.

16. Scanvorrichtung nach einem der vorhergehenden Ansprüche, wobei jede Scaneinheit ein Winkel-Sichtfeld (AFOV) zwischen 60° und 75° definiert.

17. Scanvorrichtung nach einem der vorhergehenden Ansprüche, wobei für eine gegebene Scaneinheit der Mittelpunkt-zu-Mittelpunkt-Abstand zwischen der Projektoreinheit und einer gegebenen Kamera dieser Scaneinheit zwischen 2 und 5 mm, vorzugsweise zwischen 3 und 4 mm beträgt.

18. Scanvorrichtung nach einem der vorhergehenden Ansprüche, wobei das Sichtfeld jeder Scaneinheit mindestens 400 mm² bei einem Arbeitsabstand zwischen 15 mm und 50 mm beträgt.

19. Scanvorrichtung nach einem der vorhergehenden Ansprüche, wobei die Scanvorrichtung ein triangulationsbasiertes Scanprinzip verwendet.

20. Scanvorrichtung nach einem der vorhergehenden Ansprüche, wobei die Scanvorrichtung einen oder mehrere Prozessoren umfasst, die zur Erzeugung einer dreidimensionalen (3D) Darstellung des dentalen Objekts eingerichtet sind.

## Revendications

1. Dispositif de balayage intra-oral (1) pour le balayage d'un objet dentaire (2), comprenant :
- une sonde allongée (3) définissant un axe longitudinal (4) du dispositif de balayage (1) ;
- une première unité de balayage (14) comprenant :
- une première unité de projecteur configurée pour projeter un motif lumineux (7) sur une surface de l'objet dentaire (2), dans lequel la première unité de projecteur définit un premier axe optique de projecteur ;
- au moins une caméra (9) comprenant un capteur d'image (10) pour l'acquisition d'images, dans lequel l'au moins une caméra (9) définit un axe optique de caméra (11) ; et
- un élément réfléchissant (12) configuré pour réfléchir la lumière provenant de la première unité de projecteur et/ou réfléchir la lumière provenant de la surface de l'objet dentaire (2) et sur l'au moins un capteur d'image (10) de chaque caméra (9) ;
- une première unité de balayage (16) comprenant :
- une seconde unité de projecteur configurée pour projeter un motif lumineux (7) sur une surface de l'objet dentaire (2), dans lequel la seconde unité de projecteur définit un second axe optique de projecteur ; et
- au moins une caméra comprenant un capteur d'image pour l'acquisition d'images, dans lequel l'au moins une caméra définit un axe optique de caméra ;
dans lequel chaque unité de balayage (14 ; 16) définit un champ de vision (FOV), **caractérisé en ce que** l'axe optique de projecteur et l'axe optique de caméra de chaque unité de balayage définissent un angle caméra-projecteur compris entre 5° et 10°.

2. Dispositif de balayage selon la revendication 1, dans lequel l'unité de projecteur est configurée pour projeter une lumière blanche non polarisée.

3. Dispositif de balayage selon l'une quelconque des revendications précédentes, dans lequel le motif lumineux est statique dans le temps.

4. Dispositif de balayage selon l'une quelconque des revendications précédentes, dans lequel le dispositif de balayage comprend une fenêtre optique faite d'un polymère ou de verre, dans lequel la fenêtre optique est située dans une extrémité distale de la sonde allongée.

5. Dispositif de balayage selon l'une quelconque des revendications précédentes, dans lequel les première et seconde unités de balayage sont agencées pour projeter le motif lumineux à travers la fenêtre optique.

6. Dispositif de balayage selon l'une quelconque des revendications précédentes, dans lequel la seconde unité de balayage comprend en outre un élément réfléchissant configuré pour réfléchir la lumière provenant de la seconde unité de projecteur et/ou réfléchir la lumière provenant de la surface de l'objet dentaire et sur l'au moins un capteur d'image de chaque caméra de la seconde unité de balayage.

7. Dispositif de balayage selon l'une quelconque des revendications précédentes, dans lequel les unités de balayage sont positionnées le long de l'axe longitudinal du dispositif de balayage.

8. Dispositif de balayage selon l'une quelconque des revendications précédentes, dans lequel les premier et second axes optiques de projecteur sont sensiblement parallèles.

9. Dispositif de balayage selon l'une quelconque des revendications précédentes, dans lequel une boîte englobante minimale d'une première unité de balayage chevauche une boîte englobante minimale d'une seconde unité de balayage.

10. Dispositif de balayage selon l'une quelconque des revendications précédentes, dans lequel les première et seconde unités de balayage sont configurées pour projeter le motif lumineux sur le même côté du dispositif de balayage.

11. Dispositif de balayage selon l'une quelconque des revendications précédentes, dans lequel chaque unité de balayage comprend deux ou plusieurs caméras, dans lequel lesdites caméras sont configurées pour acquérir un ensemble d'images.

12. Dispositif de balayage selon la revendication 11, dans lequel le nombre d'images dans l'ensemble d'images correspond au nombre de caméras dans une unité de balayage donnée.

13. Dispositif de balayage selon l'une quelconque des revendications 11 et 12, dans lequel les caméras sont synchronisées de telle sorte que les images au sein de l'ensemble d'images sont acquises simultanément.

14. Dispositif de balayage selon l'une quelconque des revendications précédentes, dans lequel chaque unité de balayage comprend quatre caméras.

15. Dispositif de balayage selon l'une quelconque des revendications précédentes, dans lequel chaque unité de balayage comprend une unité de projecteur et une pluralité de caméras, dans lequel les caméras sont agencées sur deux axes pour former une croix dans le plan, dans lequel l'unité de projecteur est située au centre de la croix lorsqu'elle est observée le long de l'axe optique de projecteur, dans lequel les deux axes sont sensiblement parallèles à au moins deux bords de l'élément réfléchissant.

16. Dispositif de balayage selon l'une quelconque des revendications précédentes, dans lequel chaque unité de balayage définit un champ de vision angulaire (AFOV) compris entre 60° et 75°.

17. Dispositif de balayage selon l'une quelconque des revendications précédentes, dans lequel pour une unité de balayage donnée, la distance de centre à centre entre l'unité de projecteur et une caméra donnée de cette unité de balayage est comprise entre 2 et 5 mm, de préférence entre 3 et 4 mm.

18. Dispositif de balayage selon l'une quelconque des revendications précédentes, dans lequel le champ de vision de chaque unité de balayage est d'au moins 400 mm² à une distance de travail comprise entre 15 mm et 50 mm.

19. Dispositif de balayage selon l'une quelconque des revendications précédentes, dans lequel le dispositif de balayage met en œuvre un principe de balayage basé sur la triangulation.

20. Dispositif de balayage selon l'une quelconque des revendications précédentes, dans lequel le dispositif de balayage comprend un ou plusieurs processeurs configurés pour la génération d'une représentation tridimensionnelle (3D) de l'objet dentaire.
